(19) Europäisches Patentamt
European Patent Office
Office européen des brevets

(11) Publication number : **0 616 814 A1**

(12) **EUROPEAN PATENT APPLICATION**

(21) Application number : **94400608.9**

(22) Date of filing : **21.03.94**

(51) Int. Cl.$^5$ : **A61L 27/00,** A61L 25/00, A61K 37/02

(30) Priority : **26.03.93 US 37786**

(43) Date of publication of application : **28.09.94 Bulletin 94/39**

(84) Designated Contracting States : **AT BE CH DE DK ES FR GB GR IE IT LI LU MC NL PT SE**

(71) Applicant : **Bristol-Myers Squibb Company**
**345 Park Avenue**
**New York, N.Y. 10154 (US)**

(72) Inventor : **Gombotz, Wayne, Richard**
**6406 N.E. 129 Place**
**Kirkland, Washington 98034 (US)**
Inventor : **Bouchard Lisa, Servito**
**2223 Benson Rd. So. T-202**
**Renton, Washington 98055 (US)**
Inventor : **Pankey, Susan Carol**
**3616 45th West**
**Seattle, Washington 98199 (US)**
Inventor : **Hawkins, Michael E.**
**2350 West 3505**
**Columbia City, Indiana 46725 (US)**
Inventor : **Purchio, Anthony F.**
**801 33rd Avenue, N.E.**
**Seattle, Washington 98133 (US)**

(74) Representative : **Durand, Yves Armand Louis et al**
**LAW OFFICES WEINSTEIN**
**20 Avenue de Friedland**
**F-75008 Paris (FR)**

(54) **Compositions for controlled release of biologically active TGF - beta.**

(57) Ceramic and polymer based compositions which direct the controlled sustained release of TGF-β to bone tissue and improved surgical implant devices incorporating such compositions are described. The compositions and improved surgical implant devices of the invention induce and/or enhance the process of osteogenesis in animal bone tissue thereby resulting in the formation of new mature bone, and are useful for repairing orthopaedic and periodontal bone tissue defects. Several particular embodiments are described by way of examples which illustrate the methods of manufacturing the compositions and improved surgical implants, the TGF-β release kinetic properties of the compositions, and the efficacy of the compositions and improved surgical implant devices in animal models. In one such particular embodiment, a critical defect introduced into a rat skull is repaired using a ceramic-based TGF-β delivery composition.

Fig. 1

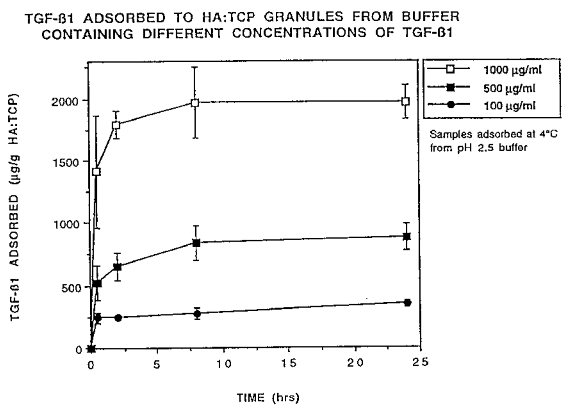


EP 0 616 814 A1

1. INTRODUCTION

The present invention is directed to compositions which provide controlled release of biologically active transforming growth factor-beta (TGF-β) to bony tissue, thereby inducing osteogenic activity and coordinating the accelerated formation of new bone. The compositions of the invention may be used alone or may be incorporated into orthopedic implant devices and used to accelerate the formation of new bone in various medical and dental applications.

2. BACKGROUND OF THE INVENTION

Bone has a remarkable capacity for regenerative growth; however, there are many clinical indications in which the bony repair process is impaired. These include situations in which there is too much bone loss for the bone to regenerate, such as skeletal deformations caused by trauma, malformation, cancer, or reconstructive cosmetic surgeries. There is a critical need for the development of implant and drug delivery technology for use in numerous clinical situations to augment or promote bone healing.

The use of autologous cartilage and bone, presently the materials of choice for skeletal augmentation and reconstruction, have several drawbacks including donor site morbidity, limited quantities of grafting material, and unpredictable implant resorption. As a result, a variety of different materials have been studied that may function as artificial implants for bone repair, including ceramics, composites, bone derivatives and natural and synthetic polymers (Damien et al., 1991, J. Appl. Biomat., 2:187-208).

An ideal implant or graft material must possess certain basic properties, including biocompatibility, sterilizability, osteoinductive activity (i.e., stimulation of phenotypic conversion of mesenchymal cells into osteoblasts, with bone formation) and osteoconductive activity (i.e., acting as a trellis for new bone formation). Porosity and pore density of the implant material play an important role in its osteoconductive properties. Implant materials should also degrade in concert with new bone growth without interfering with the formation of new bone. The implant should not induce adverse local tissue reaction, be immunogenic, or systemically toxic. Many of the materials presently under investigation only partially satisfy these requirements.

Poly(lactic acid) (PLA) and poly(lactic-coglycolic acid) (PLPG) polymers have been used experimentally for osseous repair. Both polymers are biocompatible and biodegradable and have demonstrated a capacity to induce bony wound healing in some instances (Hollinger, 1983, J. Biomed. Mater. Res., 17:71-82). Both also have the advantage of mechanical strength, which is important in the augmentation of load bearing bones. However, neither PLA nor PLPG are osteoinductive, and in some cases their lack of porosity can obstruct bone penetration into the implant (Tencer et al., 1987, J. Orthop. Res., 5:275-282).

Demineralized bone matrix (DBM) is a bone derivative that is prepared by demineralizing cadaver bone with HC. DBM has been shown to exhibit osteoinductive activity (Guterman et al., 1988, Collagen Rel. Res., 8:419-431; Reddi and Anderson, 1976, J. Cell. Biol., 69:557-572; Firschein and Urist, 1972, Clin. Orthop., 84:263-275). Clinically, DBM is used primarily for facial skeletal augmentation and reconstruction (Mulliken et al., 1981, Ann. Surg., 194:366-372). In many cases, however, bone resorption occurs with DBM implants. A recent study concluded that demineralized bone had an unacceptably high resorption rate and should only be used in cases where the implant is positioned in sites rich in primitive mesenchymal cells or bone-forming cells (Toriumi, et al., 1990, Arch. Otolaryngol Head Neck Surg., 116:676-680). Another potential problem with the use of human DBM is the possibility that it may contain transmittable viruses such as human immunodeficiency virus and hepatitis B virus (Buck, et al., 1990, Clin. Orthop. 251: 249-253).

DBM implants also lack the mechanical strength necessary for applications involving stress resistance or weight bearing capacity, such as appendicular skeletal and mandibular applications. Various materials have been combined with DBM to form composites, having improved mechanical properties and the capacity to enhance new bone formation. For example, natural composites include DBM combined with autogenous bone marrow (Green, et al., 1986, Clin. Orthop., 205:293-298; Lindholm and Urist, 1980, Clin. Orthop., 150:288-300) and DBM combined with autogenous bone (Kohler and Kreicbergs, 1987, Clin. Orthop., 218:247-258). Several synthetic composites have been made using porous polysulfone (Vamdersteenhoven and Spector, 1983, J. Biomed. Mater. Res., 17:1003-1014) and hydroxyapatite (Hopp et al., 1989, J. Orthop. Res., 7:579-584). Only limited success has been achieved with these systems.

In an effort to develop improved methods for enhancing osteogenesis, some focus has been directed toward understanding the mechanisms which regulate bone healing. Growth factors are known to regulate the cellular functions of many processes, particularly the healing of tissue. Transforming growth factor-β (TGF-β), basic fibroblast growth factor (bFGF) and platelet derived growth factor (PDGF) are several polypeptide growth factors which are central to the tissue repair process. Among these members of the TGF-β superfamily of polypeptides appear to play critical roles in the bone healing process (See, for example, Pierce et al., 1989, J. Cell Biol, 109:429-440).

The TGF-βs are 25 kD homodimeric proteins that stimulate the migration, proliferation, and matrix synthesis of mesenchymal cells. Bone is the largest reservoir of TGF-β in the body (Seydin et al., 1986, J. Biol. Chem., 261:5693-5694), and TGF-β regulates osteoclast and osteoblast activity leading to bone tissue formation (Bonewald et al., 1990, Clin. Orthop. 250: 261; Joyce et al., 1990, J. Cell Biol. 110: 2195). The in vivo application of TGF-β1 or TGF-β2 by direct injection into the subperiosteum has been shown to increase bone thickness and chondrogenesis (Joyce, et al., 1990, J. Cell Biol., 110:2195; Marcelli et al., 1990, J. Bone Miner. Res., 5:1087-1096; Mackie and Trechsel, 1990, Bone, 11:295-300). TGF-β1 has also been shown to induce bone closure of large bony defects in the skull (Beck et al., 1991, J. Bone and Miner. Res., 6:1257-1265). Recently, a member of the TGF-β superfamily of growth and differentiation regulators, bone morphogenic protein-5 (BMP-5) has been connected to normal skeletal development. Extensive deletions in the BMP-5 gene in mice translates into altered size, shape, and number of specific skeletal elements (Kingsley et al., 1992, Cell 71: 399-410).

Despite these promising findings, there exists a need for effective methods of delivering stable, bioactive TGF-βs to bony defects in order to promote bone healing and permanently repair the defect. Also, there exists a need for a method of predictably controlling the amount and duration of TGF-β release from delivery and implant systems over a given time period. Such systems should possess the biodegradability, mechanical properties, morphology, and/or porosity necessary for successful permanent osseous repair and augmentation.

## 3. SUMMARY OF THE INVENTION

The present invention is directed to compositions which are useful to deliver biologically active TGF-β to human bony tissue in order to induce osteogenesis. One aspect of the invention relates to ceramic-based compositions containing TGF-β, and to surgical implants coated or impregnated with such compositions, which may be used to facilitate osteogenesis in a variety of medical and dental applications. The ceramic-based TGF-β delivery compositions of the invention can be designed and manufactured to release biologically active TGF-β according to different release kinetics by varying the conditions under which the compositions are manufactured. This feature of the invention is particularly useful since different medical and dental applications may require compositions having different release kinetics.

The invention is further directed to biodegradable polymer-based compositions incorporating TGF-β. Biodegradable polymer-based TGF-β delivery compositions also release controllable amounts of biologically active TGF-β to bony tissue sites resulting in the induction of osteogenesis. Biodegradable polymer based TGF-β delivery compositions of the invention may be particularly useful for applications that require a flexible, malleable or semi-fluid material, rather than rigid materials such as the ceramic-based compositions of the invention. Such applications include but are not limited to the treatment of periodontal disease, small bone defect repair, and the repair of bone defects exposed to minimal mechanical stress. Biodegradable polymer-based TGF-β delivery compositions may be mixed with various polymeric gels and then applied directly to the bone defect. Such polymeric gels include synthetic materials (e.g., glycerol, methyl cellulose, hyaluronic acid, polyethylene oxide and polyoxamers) and naturally derived materials (e.g., collagen, gelatin, hyaluronic acid and alginates. Additionally, the biodegradable polymer-based compositions of the invention may be coated onto and/or impregnated into various metallic, ceramic, polymeric or other alloplastic implant materials to augment the ability of the implant to become stably integrated to bone tissue surrounding the implant site. The polymer-based and ceramic-based TGF-β delivery compositions may be combined into mixed polymer/ceramic based TGF-β delivery compositions. Such mixed compositions may be prepared by combining pre-formulated ceramic- and polymer-based compositions, or by coformulating the mixed composition.

The compositions of this invention may be particularly useful for the treatment of non-union fractures, skeletal defects, periodontal disorders and for anchoring alloplastic orthopedic implants such as artificial hips, knees, and repair hardware (e.g. screws, pins, etc.).

## 4. BRIEF DESCRIPTION OF THE FIGURES

FIG. 1. Amount of TGF-β1 adsorbed to HA/TCP granules as a function of time and the concentration of TGF-β1 in the adsorption solution.

FIG. 2. Amount of TGF-β1 adsorbed to HA/TCP granules at 4° C for 24 hours as a function of TGF-β1 concentration.

FIG. 3. Amount of TGF-β1 adsorbed to HA/TCP granules at 4° C for 24 hours from pH 2.5 and 6.5 buffers as a function of TGF-β1 concentration.

FIG. 4. Amount of TGF-β1 adsorbed to HA/TCP granules at 4° C for 24 hours from (A) pH 2.5 and (B) pH 6.5 buffers as a function of TGF-β concentration. The amount of TGF-β1 adsorbed was determined by both the solution depletion method and the direct method after washing the granules in PBS as described in Section 6.2.2., *infra*.

FIG. 5. Amount of TGF-β1 adsorbed to HA/TCP granules at 4° C for 8 hours under different adsorption conditions.

FIG. 6. Cumulative amount of TGF-β1 released from HA/TCP granules over time into PBS containing 1% BSA. Granules were adsorbed with TGF-β1 from solutions at (A) pH 2.5 or (B) pH 6.5 at 4°C for 12 hrs. The TGF-β1 concentrations were 500, 250 or 100 μg/ml.

FIG. 7. Total percent of TGF-β1 released from HA/TCP granules onto which different amounts of TGF-β were adsorbed after being placed in PBS with 1% BSA at 37°C for 340 hrs.

FIG. 8. Schematic illustration of various methods for coating titanium cell culture discs with TGF-β1.

FIG. 9. Amount of TGF-β1 adsorbed to HA/TCP coated and uncoated titanium cell culture discs.

FIG. 10. Amount of TGF-β1 released from cell culture discs at 37° C into PBS containing 1% bovine serum albumin.

FIG. 11. Total amount of TGF-β1 extracted from HA/TCP coated titanium implants as determined by both ELISA and GIA as described in Sections 6.1.4. and 6.1.5., infra.

FIG. 12. Cumulative amount of TGF-β1 released over time from $CaSO_4$ matrix devices loaded with different amounts of TGF-β1.

FIG. 13. Cumulative amount of TGF-β1 released over time from $CaSO_4$ matrix devices containing 250 μg TGF-β1/g device, with or without mannitol.

FIG. 14. Cumulative percent and total amount of TGF-β1 released over time from biodegradable microspheres made from various molecular weight PLPG copolymers.

FIG. 15. ELISA and GIA activity of TGF-β1 released from the 67kDa polymer based PLPG microspheres at 37° C into PBS containing 1% bovine serum albumin.

## 5. DETAILED DESCRIPTION OF THE INVENTION

The invention described herein relates to novel ceramic and polymer based compositions incorporating TGF-β ("TGF-β delivery compositions"), which are useful to deliver TGF-β to bony tissue, thereby inducing, and/or thereafter maintaining to a sufficient extent, the process of osteogenesis at the site of application, such that enhanced new bone formation is achieved. The compositions of the invention may be useful in a number of medical and dental applications for which accelerated bone healing and new bone formation is the principal object. The invention is described by way of examples describing the methods and parameters for fabricating and testing the TGF-β delivery compositions of the invention, and the specific characteristics and effectiveness of various ceramic and polymer based TGF-β delivery compositions and surgical implant systems.

### 5.1. PREPARATION OF TGF-β DELIVERY COMPOSITIONS

The TGF-β delivery compositions of the invention may be prepared using the techniques described in the example sections which follow, or by other techniques available to those in the art which result in similar formulations capable of releasing TGF-β and inducing and/or maintaining osteogenesis.

The TGF-β delivery compositions of the invention are capable of delivering sustained amounts of TGF-β to a bone tissue application site in an animal for extended time periods, and thereby enhancing osteogenesis and new bone tissue formation in the application site, and comprise a biodegradable ceramic and/or polymer carrier material into which TGF-β is incorporated, at concentrations of between 1 ng and 1 mg per gram carrier material. As used herein, "delivering sustained amounts of TGF-β" means delivering a quantity of TGF-β over a period of time which is greater than the quantity of TGF-β which can be delivered by a single, conventional administration route (e.g., injection). As used herein, the term "bone tissue" refers to any bony tissue, including bone tissue of osteoblastic, chondroblastic and ondotoblastic origin. Any of the TGF-β isoforms and related molecules may be used in the formulation of the TGF-β delivery compositions of the invention described herein, including but not limited to homodimeric forms of TGF-β1, TGF-β2, TGF-β3, TGF-β4, TGF-β5, bone morphogenic proteins, etc., and heterodimers thereof. The mature, biologically active form of the TGF-β molecule is preferred, although other forms of TGF-β (e.g., latent high molecular weight TGF-β) and structurally modified or hybrid TGF-βs (e.g., TGF-5β; Madisen et al., 1990, Ann. N.Y. Acad. Sci. 593:7-25) which induce osteoinduction at the desired site following release from the carrier material may also be used. Recombinant, naturally isolated or synthetic TGF-βs may be utilized equally well. Methods for the preparation of recombinant TGF-βs are described in co-owned and copending United States Patent Application Serial Nos. 07/958,522, filed October 8, 1992, 07/667,246, filed March 8, 1991, 07/669,171, filed March 14, 1991, and 07/446,020, filed December 5, 1989, each of which is incorporated herein in its entirety. As used herein, the term "TGF-β" includes all naturally-occurring homodimeric and heterodimeric isoforms of TGF-β, structurally modified and hybrid TGF-βs (e.g. TGF-5β), mature and precursor forms thereof, and engineered heterodimers thereof.

A variety of techniques, carrier materials and additives may be used for formulating of the TGF-β delivery compositions of the invention. Such techniques include but are not limited to the direct adsorption of TGF-β onto ceramic, polymeric or other alloplastic materials, the incorporation of TGF-β into such materials to form three dimensional matrices such as films, slabs, microspheres or gels, or the application of such TGF-β-incorporating materials into surgical implants.

Formulations of various TGF-β delivery compositions are described by way of the examples presented in Sections 6 through 10, *infra,* in which biologically active recombinant mature TGF-β1 is used as the TGF-β component. Formulations of ceramic-based TGF-β1 delivery compositions are described in Sections 6 and 8, *infra,* and their use to modify surgical implants is described in Sections 7 and 10, *infra.* Formulations of polymer-based TGF-β1 delivery compositions are described in Section 9, *infra.*

Ceramic-based TGF-β delivery compositions comprise a ceramic carrier material and a TGF-β. Acceptable ceramic carrier materials include but are not limited to hydroxyapatite ceramics, hydroxyapatite/calcium phosphate ceramics (e.g., hydroxyapatite/tricalcium phosphate), florapatite ceramics (fluorine-substituted hydroxyapatite). Ceramic-based TGF-β delivery compositions may be formulated by, for example, adsorption of TGF-β onto the ceramic carrier material, as described in Section 6.1.2, *infra,* and by mixing TGF-β with ceramic powders, pastes, and the like. Adsorption of TGF-β onto a ceramic carrier material may be achieved by immersing and soaking the carrier material in solution formulations of TGF-β and buffer under conditions suitable for the desired composition. Preferred buffers for adsorption of TGF-β onto ceramic carrier materials include organic acid buffers such as, for example, citric acid and acetic acid, most preferably at about pH 2.5. Such buffers serve to stabilize TGF-β, thereby enhancing the amount of biologically active TGF-β adsorbed to the ceramic carrier material. Additives such as mannitol, human serum albumin, gelatin and/or non-ionic detergents may also be incorporated into the adsorption buffer in order to influence the extent of adsorption of the TGF-β component.

As illustrated in the example sections which follow, the ceramic-based compositions of the invention can be designed and manufactured to release TGF-β according to different release kinetics by varying the conditions under which the compositions are manufactured. Such conditions include but are not limited to the concentration of TGF-β used for adsorption onto or mixing with the carrier material; the adsorption or mixing buffer ingredients and pH; adsorption time; the incorporation of additives (e.g., mannitol); and the use of wash steps during manufacture. Generally, the use of higher concentrations of TGF-β in the adsorption solution will result in the incorporation of greater amounts of TGF-β into the composition.

For the manufacture of TGF-β delivery compositions having high concentrations of biologically active TGF-β, a ceramic carrier material with a high surface area to weight ratio is preferred, such as, for example hydroxyapatite-tricalcium phosphate (HA/TCP) granules (Section 6.2.1., *infra*). Adsorption time may also be used to control the amount of TGF-β incorporated into ceramic-based TGF-β delivery compositions, as the amount of TGF-β adsorbed increases upon lengthier adsorptions. Near maximal incorporation levels using adsorption solutions containing between 100 and 1000 μg/ml mature TGF-β are achieved by approximately 8 to 10 hours adsorption time.

Another variable which may be used to vary the amount of TGF-β incorporated into a ceramic carrier material is the pH of the adsorption solution. Lower pH levels (e.g., pH 2.5) appear to result in more efficient incorporation compared to higher pH buffers (Section 6.2.2., *infra*, and FIG. 3). The addition of additives may also be used to influence the extent of TGF-β adsorption onto the ceramic carrier, and hence, the TGF-β release characteristics of the resulting composition. As described in Section 6.2.3., the addition of 30 μg/ml mannitol to the adsorption buffer significantly reduced the amount of TGF-β1 adsorbed to HA/TCP granules. It is interesting to note that the use of a mannitol additive in the formulation of a calcium sulfate-based TGF-β1 delivery composition had the opposite effect (Section 8.2.1., *infra*).

The quantity of TGF-β incorporated into a TGF-β delivery composition will depend on various factors associated with the desired objective, including, for example, the nature and extent of the bony defect or site to be treated.

The quantity of TGF-β incorporated into the ceramic-based TGF-β delivery compositions will also influence release kinetics. As an example, mature TGF-β1 is released from HA/TCP-based compositions in two phases, the first characterized by a more rapid release rate in which nearly half of the incorporated TGF-β1 is released compared to the second phase (Section 6.2.4, and FIG. 6). Generally, first phase release kinetics for HA/TCP-based TGF-β1 delivery compositions are fastest during the first 25 to 50 hours (FIG. 6).

The choice of carrier material also influences the particular release kinetics a TGF-β delivery composition will have. Thus, release kinetics of a TGF-β delivery composition may be further defined during the manufacture process by choice of carrier material. For example, calcium sulfate-based compositions have noticeably different release kinetic profiles for different levels of TGF-β incorporation when compared to HA/TCP-based composition release kinetic profiles (compare FIGS. 6A and 6B with FIG. 12).

Similarly, varying the manufacture conditions of polymer-based TGF-β delivery compositions will also influence the amount of TGF-β incorporated and the release kinetic properties of the resulting compositions. Choice of polymer carrier material, for example, may dramatically influence resulting release kinetics. In this connection, the use of different molecular weight forms of the same polymer may be used to dramatically influence the release kinetic properties of the resulting compositions (Section 9.2.2., *infra*). The amount of TGF-β incorporated into poly (lactide-co-glycolide) copolymer (PLPG) microsphere-based TGF-β delivery compositions and the release rate of TGF-β therefrom can be controlled by varying the microsphere fabrication conditions. Such conditions include, for example, the TGF-β loading, the addition of additives to accelerate or inhibit release, and PLPG polymer concentration and formulation (e.g., polymer molecular weight, PLPG ratios, and lactide/glycolide distribution in the polymer chain).

Various polymer carriers known in the art may be utilized as carrier materials for formulating polymer-based TGF-β delivery compositions, in addition to the PLPG copolymers and polyoxamer gel based TGF-β1 delivery compositions described in the examples which follow, including but not limited to poly(lactic acid), poly(glycolic acid), poly(caprolactone), poly(hydroxybutric acid), poly(orthoesters), and other biodegradable polyesters, poly(anhydrides), poly(carbonates), poly(amides), poly(acetals), and poly(cyanoacrylates), (e.g. poly(trimethylene carbonate)).

5.2. EVALUATION OF OSTEOGENIC CAPACITY

TGF-β delivery compositions and surgical implants incorporating such compositions may be characterized for their release kinetics and/or osteoinductive activity using various *in vitro* assays known in the art for assaying TGF-βs, including various immunological techniques and TGF-β bioassays, such as the TGF-β ELISA and growth inhibitory assays described in Sections 6.1.3., and 6.1.4., respectively, *infra.* Release kinetics may be determined by any assay which is capable of detecting TGF-β. Immunological assays such as, for example, Western blots, radioimmunoprecipitation, and enzyme-linked immunoassays may be used to detect and quantitate TGF-β incorporated into and released from TGF-β delivery compositions and implants. (TGF-β monoclonal antibody detection kits are available from R&D Systems, Inc., Minneapolis, MN). *In vitro* assays for detecting biologically active TGF-β include those which detect specific binding to TGF-β receptors and those which measure a biological activity of TGF-β, such as osteoinductive activity, growth inhibitory activity, etc. A widely used *in vitro* bioassay which measures growth inhibition of mink lung cells responsive to TGF-β is described in Section 6.1.5., *infra.*

TGF-β delivery compositions and orthopedic implants incorporating such compositions may also be tested for their capacity to induce osteoinduction and new bone formation *in vivo.* Although the assays systems for such *in vivo* evaluations are potentially as numerous as the various applications for which the compositions of the invention may be used, several well established models for the evaluation of bone healing and repair have been developed, such as critical defect, non-union fracture, and humeral gap animal models. (See, for example, Schmitz et al., 1986, Clin. Ortho. & Related Res. 205:299-308). A particular embodiment of a critical defect model is described in Section 8.2.2., *infra*. A canine humeral non-weight-bearing gap model is described in Section 10.1., *infra*. Animal models which may be used to evaluate the capacity of a TGF-β delivery composition to induce periodontal regeneration are known(Lynch et al., 1991, J. Periodontol. 62:458-467).

5.3. IMPROVED SURGICAL IMPLANTS

A further aspect of the invention is directed to the use of the TGF-β delivery compositions described herein to create improved surgical implant devices. Such devices include but are not limited to those routinely employed in orthopaedic and periodontal surgery to connect, reform, and supplement skeletal tissues and/or correct various structural defects.

In one embodiment of this aspect of the invention, surgical implants such as, for example, metallic screws, pins, and artificial hips, are modified to incorporate a TGF-β delivery composition or a combination of TGF-β delivery compositions. Such modified surgical implants may have improved apposition and/or anchoring characteristics relative to unmodified implants since they will release sustained amounts of biologically active TGF-β to the part(s) of the recipient's bone to which the implant is directly connected, thereby resulting in osteoinduction of the adjacent tissue, and ultimately in the formation of hard bone stably integrated around and through the implant. The net result is an implant that has become firmly affixed to the implant site and permanently integrated into the recipient's skeleton.

*In vivo* efficacy of metallic surgical implants modified to incorporate a ceramic-based TGF-β1 delivery composition is described by way of example in Section 10., *infra.* The modified implants were implanted into surgically introduced gaps in canine humeri, where they induced osteogenesis, new bone formation, and became affixed to the implant site.

The TGF-β delivery compositions of the invention may be used to modify surgical implants, using the techniques described herein, as well as other techniques which will become apparent to those skilled in the art. For example, hydroxyapatite/tricalcium phosphate-based TGF-β delivery compositions may be fabricated and used to modify metallic surgical implants such as titanium and porous titanium fiber coated metallic implants. As described in Section 7., *infra,* metallic surgical implants coated with hydroxyapatite/tricalcium phosphate and further modified to incorporate recombinant TGF-β1 are capable of releasing biologically active TGF-β1 over sustained periods. Similarly, as also described in Section 7., metallic surgical implants may be modified to integrate ceramic and polymer based TGF-β1 delivery compositions, resulting in an improved implant capable of releasing biologically active TGF-β1 over sustained periods.

The choice of TGF-β composition may be influenced by the particular surgical result desired. For example, the optimal incorporation of one type of implant device may require the release of a high concentration of TGF-β over a relatively brief time frame, whereas optimal incorporation of another type of implant may require sustained release of lower concentrations of TGF-β over a longer period. In the former situation, TGF-β compositions based on polyoxamer gels and the like may be most appropriate, since TGF-β is quickly released in this delivery system. In the latter situation, the requirement for sustained slow release may be best met by the use of implant devices modified to integrate ceramic-based TGF-β delivery compositions, such as the calcium phosphate/TGF-β composition-modified titanium implants described in Section 7., *infra.*

In another embodiment of this aspect of the invention, biodegradable surgical implants capable of establishing an osteoinductive process leading to permanent bone formation may be fabricated from the ceramic based TGF-β delivery compositions of the invention and used in surgical applications requiring rapid repair of critical bone defects, non-union fractures, and other bony defects. In a specific embodiment, described by way of example in Section 8., *infra,* cylindrical implants fabricated with calcium sulfate and recombinant TGF-β1 are used to induce osteoinduction, new bone formation, and the initiation of complete repair of a 9 millimeter diameter hole introduced into the skull of experimental rats. Visual examination of the defect area 6 weeks after insertion of the implant revealed hard, new bone covering the majority of the defect which was barely distinguishable from the surrounding bone tissue. Microradiographic analysis was used to visualize the quantity of mineralized new bone in the defect. Histologic examination of this new bone tissue revealed that more new bone was formed in implants incorporating higher concentrations of TGF-β (See Table III, *infra).* None of the ceramic/TGF-β composition remained in the defect area, indicating that the implant had completely degraded over time without interfering with the new bone formation process it had induced.

## 5.4. PERIODONTAL APPLICATIONS

Yet another aspect of the invention relates to methods and compositions for the treatment of periodontal disease. TGF-β delivery compositions and dental implants which incorporate such compositions or which are fabricated directly from such compositions may be useful to enhance periodontal regeneration. Periodontal disease is caused by various species of bacteria which colonize the surfaces of tooth roots. Severe destruction of the gingiva, the periodontal ligament, and the alveolar bone is the result, leading ultimately to tooth loss.

At present, treatment of periodontal disease has met with limited success. The goal of current therapies is the regeneration of the periodontal tissues - cementum periodontal ligament and alveolar bone. Successful therapy has been particularly elusive in advanced cases of bone destruction. A recently published report describes the use of a combination of platelet-derived growth factor and insulin derived growth factor, formulated in a methylcellulose gel, to successfully enhance the formation of new bone and cementum in dogs with periodontal disease (Lynch et al., 1991, J. Periodontol. 62:458-467).

The TGF-β delivery compositions described herein may be useful to induce periodontal regeneration over a period of time sufficient to allow new cementum and bone formation. Sustained release of TGF-β1 may be important for successfully treating advanced cases of periodontal disease involving widespread and severe bone destruction. In this connection, the release kinetics of the treatment composition may be coordinated to the nature and severity of the disease during fabrication of the TGF-β delivery composition.

Surgical implants comprising TGF-β delivery compositions may be particularly useful as single tooth replacements and implants which can be used to anchor crowns and bridges. At present, single tooth implants do not consistently support the effective integration of surrounding alveolar bone. TGF-β delivery compositions incorporated into such implants may stimulate the integration of the alveolar bone, thereby resulting in a fully integrated and stable dental implant. Similarly, the placement of biodegradable TGF-β delivery compositions into the implant site may stimulate the interaction of the alveolar bone and the implant itself.

### 5.5. STORAGE FORMULATIONS

The ceramic and polymer based TGF-β delivery compositions described herein may be lyophilized for long term storage without substantial loss of TGF-β biological activity.

## 6. EXAMPLE: PREPARATION AND *IN VITRO* EVALUATION OF CERAMIC-BASED TGF-β1 DELIVERY COMPOSITIONS

### 6.1. MATERIALS AND METHODS

#### 6.1.1. TGF-β1 FORMULATION

Purified recombinant simian TGF-β1 in mature form was prepared essentially as described in co-owned and copending United States Patent Application Serial No. 07/958,522, filed October 3, 1992, which is incorporated by reference herein in its entirety, and formulated into a 30mM citric acid or sodium phosphate buffer solution at various pH levels and containing various concentrations of TGF-β1 and other additives as indicated in the results sections which follow.

#### 6.1.2. HA/TCP GRANULES AND ADSORPTION CONDITIONS

Hydroxyapatite/Beta tricalcium phosphate (HA/TCP) granules having a diameter of 0.5 to 1.0 mm and a surface area to weight ratio of 1.29 $m^2/g$ were obtained from Zimmer, Inc., (Warsaw, Indiana) in sterile form and were stored dry at 25°C. To adsorb TGF-β, different formulations and experimental conditions were used as described in the results sections which follow. Generally, the HA/TCP granules were immersed and allowed to soak in the TGF-β formulations at 4 °C for between 30 minutes and 24 hours. In some instances, a wash step followed the adsorption procedure.

#### 6.1.3. *IN VITRO* RELEASE ASSAY

The tyrosine residues of recombinant TGF-β1 were radiolabelled with $^{125}I$ using the chloramine-T method essentially as described (Frolik et al., 1984, J. Biol. Chem. 10:10995). Radiolabelled TGF-β1 was added to unlabeled TGF-β1, resulting in TGF-β1 preparations having specific activities of 30,000 to 100,000 cpm/μg TGF-β1, which were used to prepare the TGF-β1 delivery compositions as described herein.

The TGF-β1 delivery compositions were immersed in 1 to 10 ml PBS (pH 7.4) containing 1% HSA (Sigma Chemical) to prevent adsorption to surfaces of pipette tips and vials, and incubated with gentle shaking at 37° C. At various time points, the PBS buffer was removed and replaced with fresh buffer. The quantity of TGF-β1 released was then measured by detecting radiolabel in aliquots of the removed buffer using a gamma radiation counter.

#### 6.1.4. TGF-β ELISA

Immulon II 96-well microliter plates were coated with 0.25 μg/ml of a murine anti-TGF-β monoclonal antibody from Bristol-Myers Squibb (in-house preparation) in coating buffer at pH 9.6. Each well contained 100 μl and was kept at 4°C overnight. The coating buffer was removed from the plates and blocked with 300μl/well of PTB buffer (PBS with 1% BSA and 0.5% Tween-20) for at least 1 hour. Samples and the standard were diluted in the range of 5 and 3.75 ng/ml. The predilutions were placed in the top row of a Costar transfer plate and a 2 times serial dilution of the standard and the sample were made using PTB. The PTB was shaken out of the antibody coated plate and 100 μl/well of each standard and sample dilution set were transferred to the plate. The plate was then covered with a plate sealer and stored at 4°C overnight. The samples were removed from the plates and washed three times with wash buffer (PBS with 0.05% Tween-20). To each well was added 100 μl of a 1/1000 dilution of a rabbit anti-TGF-β1 monoclonal antibody in PTB. The plate was sealed and incubated at room temperature for one hour, the reagent removed, and the plate washed three times with wash buffer. A 1/5000 dilution of goat anti-rabbit IgG-HRP in PTB was added to each well (100μl/well) and the plates were sealed and incubated at room temperature for an additional hour. After removing the reagent and washing the plate three times with wash buffer, 100μl of a 1/100 dilution of TMB in buffered substrate was added to each well. The plates were sealed and incubated at room temperature, after 15 minutes the reaction was stopped by adding 100 μl of 1.0 N sulfuric acid to each well. Absorbance was read in an automated plate reader at 450 nm.

6.1.5. TGF-β GIA

Mink lung epithelial cells, Mv 1 Lu (Accession Number CCL-64, American Type Culture Collection), which are extremely sensitive to TGF-β1 were utilized for the growth inhibition assay. The assay was performed using the thymidine analog 5'-[$^{125}$I]-ido-2'deoxyuridine ($^{125}$Idu) to assess DNA synthesis. One unit of activity was defined as the amount required to inhibit 50% incorporation of $^{125}$IdU compared to untreated CCL-64 cells. Using isolated TGF-β1 as a standard, one unit of activity generally corresponded to 80-100 pg/ml of TGF-β1.

6.2.RESULTS

6.2.1. VARIABLE ADSORPTION OF THE TGF-β ONTO HA/TCP GRANULES: TIME AND CONCENTRATION

Recombinant TGF-β1 was formulated in a 30 mM citric acid buffer containing 30 mg/ml mannitol, pH 2.5, at concentrations of 100, 500 and 1000 μg/ml. 30 mg samples of HA/TCP granules were soaked in 150 μl of the TGF-β1 solutions at 4°C for predetermined times as indicated in FIGS. 1 and 2. The enzyme linked immunosorbent assay (ELISA) described in Section 6.1.4., *supra,* was used to determine the quantity of immunoreactive TGF-β1 present in the solutions initially and after adsorption. The amount of TGF-β1 adsorbed to the HA/TCP granules was calculated by determining the quantity of TGF-β1 depleted from the initial adsorption solution.

The amount of TGF-β1 adsorbed to the HA/TCP granules as a function of time and concentration of TGF-β1 in the adsorption solution is shown in FIG. 1. The results indicate that the amount of TGF-β1 adsorbed to HA/TCP granules can be controlled by varying the TGF-β1 concentration in the adsorption solution and by varying the time that the granules are exposed to the TGF-β1 formulation. FIG. 2. shows the amount of TGF-β1 adsorbed to the HA/TCP granules as a function of TGF-β1 concentration. The fact that the curve continues to increase even at high TGF-β1 concentrations is a function of the large surface area of the HA/TCP granules and the high affinity between TGF-β1 and the HA/TCP at low pH.

6.2.2. VARIABLE ADSORPTION OF TGF-β1 ONTO HA/TCP GRANULES: pH AND CONCENTRATION

TGF-β1 was prepared in two different buffers: (i) 30 mM citric acid containing 30 mg/ml mannitol at pH 2.5, and (ii) 30 mM sodium phosphate containing 30 mg/ml mannitol at pH of 6.5. Adsorption was carried out as described in Section 6.2.1., *supra,* using TGF-β1 concentrations of 500, 250 and 100 μg/ml at 4°C for 12 hrs. The amount of adsorbed TGF-β1 was determined by the addition of a small amounts of $^{125}$I-labeled TGF-β1 as a tracer to the adsorption solution. The labeled TGF-β1 material was added to the unlabeled TGF-β1 formulations resulting in preparations having specific activities ranging from 30,000 to 100,000 cpm/μg TGF-β1. The amount of TGF-β1 adsorbed to the HA/TCP was quantitated by measuring the depletion of TGF-β1 in the adsorption solution after exposure to the HA/TCP granules, and by detecting radiolabel with a gamma counter following washing with PBS.

FIG. 3 shows the amount of TGF-β1 adsorbed to the HA/TCP granules after washing with PBS. These results indicate that more TGF-β1 is adsorbed to the HA/TCP using a pH 2.5 formulation than is adsorbed from a pH 6.5 formulation, at all TGF-β1 concentrations tested. Also, the amount of TGF-β1 adsorbed to the granules increased continually as the concentration of TGF-β1 increased, consistent with the results described in Section 6.2.1., *supra.*

The increased adsorption at lower pH may be attributable to an enhanced interaction between the TGF-β1 and the HA/TCP. Specifically, the amino groups on the TGF-β1 molecule are protonated and have a positive charge at a lower pH. These positively charged groups can ionically interact with the negatively charged phosphate groups on the HA/TCP as illustrated below.

$$HAPO_4^{-}\cdots\cdots{}^{+}NH_3\text{-TGF-}\beta$$

FIGS. 4A and 4B show the amount of TGF-β1 adsorbed to the HA/TCP granules both before and after washing the granules in PBS. The results indicate that more TGF-β1 is present on the granules prior to washing than after washing. Since the surface area/weight ratio of the HA/TCP granules (by BET nitrogen adsorption analysis) is 1.29 $m^2$/g, and the dimensions of the highly related TGF-β2 molecule are 60 Å by 20 Å by 15 Å (Daopin, et.al., 1992, Science, 257:369-373), the area occupied by each TGF-β1 molecule on the surface of the HA/TCP granules can be estimated according to the dimensions of TGF-β2. Table 1 shows the amount of TGF-β1 adsorbed/$cm^2$ surface area and the area associated with each TGF-β1 molecule on HA/TCP granules before and after washing in PBS, based on the TGF-β1 dimension estimate of 60 Å by 20 Å by 15Å. After washing, the granule surface retained approximately one TGF-β1 molecule per 3733 $Å^2$, or about three times the diameter of a single TGF-β1 molecule. Before washing the granules, however, the granule surface accommodated about one TGF-β1 molecule per 620 $Å^2$. Since this is a smaller area than a single TGF-β1 molecule, multilayer adsorption of TGF-β1 is probably occurring on the surface of the HA/TCP granules.

This example illustrates that if the HA/TCP granules are not washed, a large amount of weakly associated TGF-β1 is adsorbed to the surface. Washing the granules can therefore ultimately be used to control the amount of TGF-β1 that is associated with the HA/TCP and the rate at which the TGF-β1 is released. If an initial large amount of TGF-β1 is needed at the site of implantation over a relatively short time, the granules should be prepared without a wash step. Conversely, if smaller amounts of TGF-β1 are needed over more prolonged time periods, the granules should be washed to the appropriate extent prior to use.

**TABLE I**

**TGF-β1 ADSORBED AND SURFACE AREA OCCUPIED BY TGF-β1 ON HA/TCP GRANULES**

| ADSORPTION ($\mu g/cm^2$)[1] | | AREA/MOLECULE ($Å^2$)[2] | |
|---|---|---|---|
| PRE-WASH | POST-WASH | PRE-WASH | POST-WASH |
| .670 ± .039 | .111 ± .007 | 619.6 ± 36.5 | 3733.2 ± 252 |

[1]TGF-β was adsorbed from pH 2.5 buffer at concentration of 500 μg TGF-β per ml buffer.
[2]Area on surface of HA/TCP granules associated with a single TGF-β1 molecule. Approximate area occupied by one TGF-β2 molecule is 1200 $Å^2$. The calculation assumes that the TGF-β1 molecule has equivalent dimensions.

### 6.2.3. VARIABLE ADSORPTION OF THE TGF-β ONTO HA/TCP GRANULES: ADSORPTION CONDITIONS

TGF-β1 was prepared in 30 mM citric acid formulation buffers with or without 30 mg/ml mannitol at a pH of 2.5 and a TGF-β1 concentration of 500 μg/ml. HA/TCP granules were exposed to the different formulation buffers at 4°C for 8 hrs. One of the HA/TCP samples was subjected to a vacuum while soaking in the mannitol containing buffer to remove any trapped air that was present in the HA/TCP and thus enhance TGF-β1 adsorption. One set of granules was prewashed in a citric acid buffer containing mannitol with no TGF-β1 and then exposed to the TGF-β1 solution. This prewash was done to remove any soluble material in the granules that could potentially interfere with the TGF-β1 adsorption. The amount of TGF-β1 adsorbed to the HA/TCP was determined by using $^{125}$I-labeled TGF-β1 as a tracer and counting the radiolabel on granules directly.

FIG. 5 shows the amount of TGF-β1 adsorbed to the HA/TCP granules under variable adsorption conditions, as described above. The data illustrate that more TGF-β1 is adsorbed onto the granules if the formulation buffer does not contain mannitol. It is possible that mannitol may compete for adsorption sites on the HA/TCP. Prewashing the granules or pulling a vacuum had no effect on the subsequent amount of TGF-β1 adsorbed to the granules. These results illustrate that the amount of TGF-β1 adsorbed onto HA/TCP granules may be controlled by incorporating mannitol into the TGF-β1 formulation buffer.

### 6.2.4. SUSTAINED RELEASE OF TGF-β FROM HA/TCP GRANULES

A TGF-β1 delivery composition consisting of HA/TCP granules incorporating TGF-β1 were prepared as described in Section 6.2.2., *supra*. The composition was washing with PBS and then placed in 1 ml of PBS containing 1% BSA, and incubated at 37°C while shaking. At specified times, the buffer was removed, saved, and replaced with new buffer. The amount of TGF-β1 released over time was determined by detection of radiolabel in the retained buffer on a gamma counter.

FIGS. 6A and 6B show the amount of TGF-β1 released from the composition as a function of time for samples containing different amounts of TGF-β1. These results show an initial fast release of TGF-β1 from the composition over the first 100 hours, after which time the amount released decreases progressively. FIG. 7 shows that between 45% and 50% of the total absorbed TGF-β1 is released from the HA/TCP granules after 340 hrs. The remaining 50% of the TGF-β1 remains bound to the HA/TCP and is released more slowly over time.

Therefore, TGF-β1 is released from the HA/TCP granule-based TGF-β1 delivery composition in two phases. In the first phase, lasting approximately 100 hours, nearly half of the adsorbed bioactive TGF-β1 is released. In the second phase, a much slower and sustained release of the adsorbed TGF-β1 occurs.

## 7. EXAMPLE: PREPARATION AND *IN VITRO* EVALUATION OF SURGICAL IMPLANTS INCORPORATING CERAMIC-POLYMER-BASED TGF-β1 DELIVERY COMPOSITIONS

### 7.1. MATERIALS AND METHODS

Metallic culture discs (22mm diameter, 3mm thick) of titanium alloy (Zimmer, Inc.) were plasma coated on one side with HA/TCP granules. The disks were sterilized by gamma irradiation and stored at 25°C. The humeral bone implants (Zimmer, Inc.) were made from a 1.5 mm thick cylindrical fiber metal sleeve of 50% porosity 30 mm long and 7 mm in outer diameter, sintered onto a Ti6A14V core rod. The implant was plasma spray coated with HA/TCP essentially as follows. Plasma spraying techniques are well known in the art (see, for example, Bunshah, Deposition Technologies for Film and Coatings, Noyes Publications, Park Ridge, New Jersey). Briefly, an electric arc is generated between two electrodes, which have a gas stream flowing between them, in a glass chamber. The gas is ionized, resulting in extremely high temperature, up to 30,000 °K. The velocity of gas stream increases to approximately the speed of sound due to the expansion of gas at such high temperature. Ceramic powder is fed into the gas stream where it is partially melted and propelled toward the target. Coating thickness on the metallic implant target is controlled by the powder feed rate. Coating thickness ranged between about 51 and 127 microns with a composition of 80 ± 15% hydroxyapatite. the balance of the coating consists of calcium phosphates and about 5% unknowns.

### 7.2. RESULTS

#### 7.2.1. ADSORPTION OF TGF-β1 ONTO AND EXTRACTION FROM HA/TCP-COATED AND UNCOATED TITANIUM DISCS

Titanium cell culture discs (FIG. 8) were either coated with HA/TCP by plasma spraying as described in Section 7.1., *supra,* or used uncoated. Each disc was placed in 0.5 ml of TGF-β1 solutions containing 1000 μg/ml recombinant TGFβ1, in a pH 2.5 or pH 6.5 buffer, prepared as described in Section 6.2.2., *supra,* for 24 hrs at 4°C. The discs were then removed from the TGF-β1 solutions, blotted on a paper towel to remove excess liquid and extracted in 4 M guanidine hydrochloride. After the extract was dialyzed against 0.2 M acetic acid, the amount of TGF-β1 in the samples was quantitated using the ELISA described in Section 6.1.4, *supra.*

FIG. 9 shows the amount of TGF-β1 that was extracted from the discs. Little to no TGF-β1 was recovered from the uncoated titanium discs. The results indicate, however, that HA/TCP TGF-β1 coated discs had TGF-β1 adsorbed to their surface. As with the HA/TCP granules, more TGF-β1 was adsorbed to the HA/TCP coating from the buffer at pH 2.5 than at pH 6.5. These results demonstrate that the adsorption of TGF-β1 to an implant can be enhanced by coating the implant with HA/TCP.

#### 7.2.2. INTEGRATION OF CERAMIC AND POLYMER BASED TGF-β DELIVERY COMPOSITIONS INTO TITANIUM IMPLANT DEVICES

TGF-β1 was also incorporated into several different carriers which were subsequently used to integrate the TGF-β1 onto titanium cell culture discs coated with HA/TCP. In one experiment, the TGF-β1 was adsorbed directly onto the HA/TCP coated disc as described in Section 7.1, *supra.* After the adsorption, one disc was stored at 4°C while one disc was frozen and lyophilized. In another experiment, TGF-β1 was mixed with a polyoxamer gel (Schmolka, 1972, J. Biomed. Mat. Res. 6:571-582) which was then applied to the discs. One polyoxamer coated disc was frozen at -70°C while the another was lyophilized. In yet another experiment, TGF-β1 was mixed with calcium sulfate ($CaSO_4$) in a 30 mM citric acid buffer with 30 mg/ml of mannitol at a pH of 2.5. The resulting paste was applied to the disc. TGF-β1 was extracted from all discs in 4M guanidine hydrochloride. The amount of TGF-β1 in the extract solutions was assayed for concentration using the ELISA described in Section 6.1.4., *supra* and for TGF-β1 bioactivity using the growth inhibitory assay (GIA) described in Section 6.1.5., *supra*. The results are provided in Table II, below.

| TABLE II | | | |
|---|---|---|---|
| AMOUNT OF TGF-β EXTRACTED FROM MODIFIED TITANIUM IMPLANTS | | | |
| SAMPLE TREATMENT | THEORETICA L LOADING (μg TGF-β1) | TGF-β1 RECOVERED BY ELISA (μg) | TGF-β1 RECOVERED BY GIA (μg) |
| Adsorption 4°C | 500 | 152 | 66 |
| Adsorption lyophilized | 500 | 105 | 125 |
| Polyoxamer 70°C | 500 | 176 | 210 |
| Polyoxamer lyophilized | 500 | 254 | 80 |
| $CaSO_4$ 4°C | 500 | 32 | 26 |

Bioactive TGF-β1 was recovered from all discs. The largest amount of TGF-β1 was extracted from the discs using the polyoxamer carrier, while the smallest amount of TGF-β1 was recovered from the disc coated with $CaSO_4$ (most likely the consequence of inefficient extraction from the $CaSO_4$). Lyophilized compositions retain at least a significant if not equivalent degree of TGF-β1 biological activity.

A set of discs treated with the same TGF-β1 compositions as those described above were evaluated for TGF-β1 release characteristics in PBS containing 1% BSA at 37°C. The amount of TGF-β1 in the releasing buffer was assayed with the ELISA described in Section 6.1.4., supra, and plotted as a function of time. The results (FIG. 10) indicate that the polyoxamer coated samples released TGF-β1 the fastest, while the $CaSO_4$ coated samples release the TGF-β1 the slowest.

These results indicate that TGF-β1 can be immobilized in a biologically active form onto HA/TCP coated titanium implants by simple adsorption or by employing ceramic or polymeric carriers. The results further show that the way in which TGF-β1 is immobilized can be used to vary the TGF-β1 release profiles, and that the implants can be lyophilized without destroying the bioactivity of the bound TGF-β1.

7.2.3. ADSORPTION OF TGF-β1 ONTO AND EXTRACTION FROM HA/TCP COATED TITANIUM HUMERAL IMPLANTS

TGF-β1 was adsorbed onto porous titanium humeral implants coated with HA/TCP as described in Section 7.1, *supra*. Briefly, the implants were placed in different solutions containing TGF-β1 (Section 6.2.2., *supra*). The implants were lyophilized and the TGF-β1 was then extracted using the procedure described in Section 7.1.1., *supra*. The amount of TGF-β1 extracted from the implants was determined by both ELISA (Section 6.1.4.) and GIA (Section 6.1.5.).

FIG. 11 shows that TGF-β1 was successfully adsorbed to all of the implants in biologically active form. Lyophilization did not appear to significantly affect TGF-β1 activity. As the concentration of TGF-β1 in the adsorption solution was increased, the amount of TGF-β1 adsorbed to the implant also increased. In general, for a given solution concentration of TGF-β1, more TGF-β1 adsorbed to the implants at pH 2.5 than at pH 6.5. This result is consistent with the adsorption of TGF-β to HA/TCP granules (FIG. 3). This example illustrates that porous titanium implants can be modified to incorporate an effective TGF-β delivery composition comprising HA/TCP and TGF-β1, and that such modified implants can be lyophilized without substantial loss of TGF-β1 biological activity.

8. EXAMPLE: PREPARATION, *IN VITRO* AND *IN VIVO* EVALUATION OF SURGICAL IMPLANTS OF CALCIUM SULFATE BASED TGF-β1 DELIVERY COMPOSITIONS

8.1. MATERIALS AND METHODS

8.1.1. CALCIUM SULFATE/TGF-β IMPLANTS

Calcium sulfate ($CaSO_4$) powder was mixed with aqueous buffers containing TGF-β1 (with $^{125}I$-labeled TGF-β1 tracer) in a ratio of 1.28 g $CaSO^4$/ml solution. A 30 mM citrate buffer, pH 2.5, with or without 30 mg/ml mannitol, was used to form a ceramic paste, which was then poured into molds and allowed to harden at room temperature overnight. The amount of TGF-β1 in the resulting devices was controlled by varying the concentration of TGF-β1 in the buffer used to hydrate the ceramic powder. Devices were fabricated which contained 60, 250 and 480 μg TGF-β1/g device. TGF-β1 release kinetics were evaluated *in vitro* as described in Section 6.1.3., *supra.*

8.2. RESULTS

8.2.1. *IN VITRO* EVALUATION

FIG. 12 shows that as the TGF-β1 loading was increased, the total amount of TGF-β1 released from the devices also increased. The $CaSO_4$-based TGF-β1 delivery implants were capable of sustaining the release of TGF-β1 for over 150 hrs. Additionally, the release kinetics were affected by the addition of mannitol to the aqueous solution used to hydrate the $CaSO_4$ powder (FIG. 13). In contrast to the effect of mannitol on TGF-β1 release in the HA/TCP-based implants (Section 6.2.3., *supra),* the addition of mannitol enhanced TGF-β1 release from $CaSO_4$-based implants.

8.2.2. *IN VIVO* EVALUATION

Calcium sulfate powder was mixed in citrate buffer with 30 mg/ml mannitol, pH 2.5 as described in Section 6.2.3., *supra.* Three different sets of $CaSO_4$-based implants were made: (1) control implants with no TGF-β1, (2) implants containing 100 μg TGF-β1/g implant and (3) implants containing 780 μg TGF-β1/g implant. The resulting cylindrical implants were approximately 1 mm thick and 8 mm in diameter.

A critical defect model was used for *in vivo* evaluation of the cylindrical implants as follows. Eight adult male Sprague Dawley rats were placed under quarantine for approximately one week prior to beginning the study. The animals were divided into three groups. One group of three animals received the implants containing 100 μg TGF-β1/g implant on one side of the cranium and the control implants on the other side. Another group of three rats received the implants containing the 780 μg TGF-β1/g implant along with contralateral controls. The third group consisted of two control animals. One rat received no implant on one side and a control implant on the other side. The other rat had the excised piece of skull replaced on one side and received no implant on the other side.

Anesthesia was administered to the rats by intramuscular injection of ketamine. A 2 cm incision was made in the supraorbital area and the skin pulled back. A 9 mm diameter hole saw was used to remove two sections of the skull in each animal, one on each side of the midline. The implants were placed into the defects and the incision closed. The animals were sacrificed after six weeks with a ketamine overdose. The implants were harvested intact in continuity with the adjacent cranium and examined by direct visualization, contact microradiography and histological analysis.

All defects containing control implants or no implant were observed to be covered with soft fibrous connective tissue upon visual analysis. A small amount of bone ingrowth into the defect was observed. Thus, some healing did occur but the defects were far from being completely closed. This result indicates that the untreated defects are unable to heal completely after 6 weeks. The defect which contained the replaced piece of the animals skull was completely healed, though the outline of the defect was still clearly visible. The defects treated with implants containing the low dose of TGF-β1 showed some new bone formation, while the defects treated with implants containing the high dose contained hardened bone covering the entire defect. There was no healing in the contralateral control defects indicating that there was no carry over of TGF-β1 from one side of the skull to the other. These results demonstrate that the $CaSO_4$ implants are capable of releasing TGF-β1 in a localized area over a period of time and to an extent necessary to induce and maintain the osteogenesis process and significantly enhance healing of the defect. No remaining $CaSO_4$ was observed in the defects in any of the animals, indicating that the implant was completely resorbed without interfering with the process of new bone formation.

Table III shows the results of the microradiographic analysis, a technique which enables visualization of calcified bone. The defect treated with replaced autogenous calvarial bone exhibited the most bone formation (87.5%), as expected, compared to the control. Defects treated with implants containing the high doses of TGF-β also had a significant amount of bone present. Histological analysis of the treated defects indicated that treatment with implants containing the high dose of TGF-β1 consistently induced mineralization and new bone formation. This study was carried out for six weeks. It is anticipated that over a longer time period, more bone growth would occur in the defects.

| TABLE III | |
|---|---|
| PERCENT CALCIFIED BONE BY MICRORADIOGRAPHIC ANALYSIS | |
| IMPLANT | % BONE ± SD |
| Autogenous calvarial bone | 87.5 ± 7.5 |
| $CaSO_4$ control | 23.3 ± 6.2 |
| $CaSO_4$ low dose TGF-β1 | 20.0 ± 5.0 |
| $CaSO_4$ high dose TGF-β1 | 67.5 ± 7.5 |

This example illustrates the utility of using a ceramic implant for the localized delivery of bioactive TGF-β1 to the site of a bony defect to successfully induce osteogenesis.

## 9. EXAMPLE: PREPARATION AND *IN VITRO* EVALUATION OF BIODEGRADABLE POLYMER-BASED TGF-β1 DELIVERY COMPOSITIONS

### 9.1. MATERIALS AND METHODS

Biodegradable polymer microspheres containing TGF-β were prepared using a water/oil/water emulsion technique. Three different 50:50 poly (lactide-co-glycolide) (PLPG) copolymers were used in the study. A high molecular weight polymer (Birmingham Polymer, Inc., inherent viscosity of 0.61dl/g, average mw of 67kDa), an intermediate molecular weight polymer (inherent viscosity of 0.193dl/g, average mw of 20kDa), and a low molecular weight PLPG (Wako Pure Chemical Industries, Inc., inherent viscosity of 0.13dl/g; average mw of 10kDa). All polymers were dissolved in HPLC-grade methylene chloride at a concentration of 25% (w/v). To 6 ml of this oil phase was added 1200 µl of 23% (w/v) bovine serum albumin in citrate buffer, pH 2.5, containing 400 µg TGF-β1. The solution was emulsified with an ultrasonic probe on ice for 1 minute. The resulting emulsion was then poured into 200 ml of PBS containing 0.1% polyvinyl alcohol and homogenized for 5 minutes at room temperature. After rotovaping (300 Torr) the sample for one hour at 37°C, it was centrifuged. The supernatant was decanted and the resulting microspheres were resuspended in a small volume of water and lyophilized. The final spheres ranged in diameter from 10 to 100 µm with pore sizes of $\leqq$1µm as determined by scanning electron microscopy.

### 9.2. RESULTS

#### 9.2.1. TGF-β INCORPORATION

The supernatant resulting from the final step in the fabrication process was evaluated for residual TGF-β1 protein in order to determine incorporation efficiency during fabrication. Quantitative analysis using the ELISA described in Section 6.1.4., *supra,* showed that approximately 99% of the TGF-β1 used in the process was successfully incorporated into both of the microsphere preparations. This result was unexpected since in experimental microsphere formulations with BSA protein under the same conditions, incorporation efficiencies were consistently lower, generally on the order of 30 to 80%.

#### 9.2.2. EFFICIENT SUSTAINED RELEASE OF BIOLOGICALLY ACTIVE TGF-β

The release kinetics of the polymer microsphere-based TGF-β1 delivery compositions prepared as described above were evaluated by measuring TGF-β1 released into PBS solution in a 37°C rotating incubator. Both ELISA (for TGF-β1 quantitation) and a GIA (for determining TGF-β1 bioactivity) were used to detect released TGF-β1 protein. The release kinetics from different molecular weight preparations are shown in FIG. 14. All polymer microsphere-based TGF-β1 delivery compositions are capable of sustaining the release of biologically active TGF-β1 for a period of several weeks. However, the molecular weight of the PLPG used in the fabrication of the TGF-β1 delivery composition has a significant effect on the TGF-β release kinetics of the composition. Specifically, the compositions fabricated with microspheres of the higher molecular weight PLPG release about 5% of their total incorporated TGF-β1 within the first day. The release is then relatively slow for a period up to about 800 hours, after which time the release kinetics accelerate. The last, accelerated, release phase is due to degradation of the PLPG structural component. The compositions fabricated with microspheres of the intermediate molecular weight PLPG release no detectable TGF-β1 for about 15 days, after which time a relatively linear release kinetic profile is observed. The compositions fabricated with microspheres of the lower molecular weight PLPG release no datectable TGF-β1 for the first 150 hours, after which time a relatively linear TGF-β1 release kinetic profile is observed. In addition, the GIA results (FIG.15) show that TGF-β1 released from these polymer microsphere-based TGF-β1 delivery compositions retain biological activity.

Distinct TGF-β1 release kinetic profiles were observed for these TGF-β1 delivery compositions, indicating that the release characteristics of biodegradable PLPG polymer-based TGF-β1 delivery compositions may be controlled during the fabrication process by, *inter alia,* varying the molecular weight of the polymer component. In addition, the quantity of TGF-β1 incorporated into the composition may be varied in order to achieve desired release kinetics.

## 10. EXAMPLE: *IN VIVO* EFFICACY OF METALLIC SURGICAL IMPLANTS INCORPORATING CERAMIC-BASED TGF-β1 DELIVERY COMPOSITIONS

### 10.1. MATERIALS AND METHODS

The capacity of titanium surgical implants modified to incorporate a ceramic-based TGF-β1 delivery composition were evaluated in a canine humeral non-weight-bearing gap healing model, essentially as described in Kienapfel et al., 1992, J. Orthopaedic Res. 10:423-433.

#### 10.1.1. IMPLANTS

Surgical implants used in the study were constructed from metallic core rods onto which a 1.5 mm thick, commercially pure titanium fiber metal porous coating had been sintered (overall dimensions - 30 mm long, 7 mm outer diameter) (Zimmer; Kienapfel et al., *supra*). Implants were modified to incorporate a ceramic-based TGF-β1 delivery composition as follows. Implants were plasma coated with HB/TCP granules as described in Section 7.1., *supra*, and in the relevant sections referred to therein. HA/TCP-coated implants were then soaked in solution formulations of TGF-β1 in 30 mM citric acid, pH 2.5, containing either 1000 yg/ml or 400 μg/ml recombinant TGF-β1 (Section 6.1.1., *supra),* overnight (approx. 17 hours) at 4° C. Implants were blotted with paper and were completely immersed in liquid nitrogen until frozen. Frozen implants were then lyophilized for 48 hours and then stored at 4° C prior to use. The amount of TGF-β1 incorporated into the implant was quantified by ELISA (Section 6.1.4., *supra)* of guanidine hydrochloride extracted TGF-β1, as described in Section 7.2.1., *supra.* Two sets of implants, containing either 1136 μg (± 65 μg s.d.) or 513 μg (± 8 μg s.d.) total incorporated TGF-β1 were obtained. Control implants were not modified with TGF-β1 or HA/TCP.

#### 10.1.2. IMPLANTATION PROCEDURE

Modified surgical implants were inserted into the left and right proximal humeri of three skeletally mature male mongrel dogs essentially as described (Kienapfel et al., *supra*) using polyethylene spacers to maintain a 3 mm gap between the implant surface and the adjacent bone. The implants containing 513 μg TGF-β1 were surgically inserted into the left humerus of all three dogs, while the implants containing 1136 μg TGF-β1 were inserted into the right humerus. The effects of control implants were studied separately using the same model.

#### 10.1.3. HISTOLOGY AND SCANNING ELECTRON MICROSCOPY

The animals were sacrificed two weeks after receiving the implant, and implants were evaluated in situ by light microscopy and backscatter electron microscopy, and the amount of mineralized bone in the gap was quantified, as described in Kienapfel et al, *supra.*

### 10.2. RESULTS

The results of the study are presented in Table IV and below. No unusual tissue reaction to the implants was observed. In each of the three specimens which had received the implants containing 513 μg TGF-β1, new mineralized bone or unmineralized osteoid had grown throughout most of the depth of the gap and had become intimately associated with the HA/TCP coating on the porous titanium fibers of the implant. In the specimens which had received the implants containing 1136 μg TGF-β1, the gaps were completely filled with osteoid and with a smaller percentage of mineralized bone relative to the lower dose implants. The observed lower percentage of mineralized bone formed in the implants incorporating the higher dose of TGF-β1 could be the result of a negative feedback mechanism induced by the presence of a high concentration of TGF-β1. Specimens which had received unmodified titanium coated implants had gaps containing only 2.5% mineralized bone and some osteoid tissue after two weeks. In contrast, the implants containing 513 μg TGF-β1 appear to have significantly enhanced osteogenesis in the gap, since as much as about 9% of the gap was occupied by mineralized bone after the two week study period. Compared to positive control specimens receiving autograft replacements in the gaps, these results demonstrate that TGF-β1 containing implants are at least as effective at inducing new bone formation as are replaced autograft tissues. Compared to allograft replacements, the implants modified to incorporate the ceramic-based TGF-β1 delivery composition are more effective at inducing the formation of new bone.

## TABLE IV

**VOLUME FRACTION OF MINERALIZED BONE WITHIN GAP AT 2 WEEKS**

| SPECIMEN | UNTREATED[1] | GAP TREATMENT ONLY[2] | | TGF-β1 IMPLANT TREATMENT | |
|---|---|---|---|---|---|
| | | AUTOGRAFT | ALLOGRAFT | 513 ± 8 μg | 1136 ± 65 μg |
| 1 | 4.2 | 8.7 | | | |
| 2 | 1.5 | 8.1 | | | |
| 3 | 2.3 | | 3.1 | | |
| 4 | 2.0 | | 2.5 | | |
| 5 | | | | 1.0 | 1.0 |
| 6 | | | | 9.1 | 4.1 |
| 7 | | | | 9.0 | 6.3 |
| mean | 2.5 | 8.4 | 2.8 | 6.4 | 3.8 |
| stand. dev. | 1.2 | 0.4 | 0.4 | 4.6 | 2.7 |

[1] Data obtained from previous study in identical model using unmodified titanium coated implants (Kienapfel et al., 1992, J. Orthop. Res. 10:423-433)

[2] Data obtained from previous study using autogenous bone grafts from the host animal and allogenic cancellous bone particles form canine femoral and tibial metaphyses.

The present invention is not to be limited in scope by the embodiments disclosed herein, which are intended as single illustrations of one aspect of the invention, and any which are functionally equivalent are within the scope of the invention. Indeed, various modifications of the invention, in addition to those shown and described herein, will become apparent to those skilled in the art from the foregoing description. Such modifications are intended to fall within the scope of the appended claims.

## Claims

1. A TGF-β delivery composition capable of delivering sustained amounts of TGF-β to a bone tissue application site in an animal, and thereby enhancing osteogenesis and new bone tissue formation in the bony defect application site, which composition comprises a biodegradable ceramic carrier material into which TGF-β is incorporated.

2. The TGF-β delivery composition according to claim 1 wherein the mature TGF-β is incorporated at a concentration of between 100 ng and 1 mg TGF-β per gram ceramic carrier material.

3. The TGF-β delivery composition according to claim 1 wherein the ceramic carrier material is hydroxyapatite/tricalcium phosphate.

4. The TGF-β delivery composition according to claim 1 wherein the ceramic carrier material is calcium sulfate.

5. The TGF-β delivery composition according to claim 1 wherein the ceramic carrier material is a combination of calcium sulfate and hydroxyapatite/tricalcium phosphate.

6. The TGF-β delivery composition according to claim 1, 2, 3, 4, or 5 wherein the TGF-β is selected from the group consisting of mature, precursor, and latent forms of TGF-β1, TGF-β2, TGF-β3, TGF-β4, TGF-β5, and bone morphogenic protein.

7. The TGF-β delivery composition according to claim 1, 2, 3, 4, or 5 wherein the TGF-β is mature biologically active TGF-β1.

8. The TGF-β delivery composition according to claim 1, 2, 3, 4, or 5 wherein the TGF-β is mature biologically active TGF-5β.

9. A TGF-β delivery composition capable of delivering sustained amounts of TGF-β to a bone tissue application site in an animal, and thereby enhancing osteogenesis and new bone tissue formation in the application site, which composition comprises a biodegradable polymer carrier material into which TGF-β1 is incorporated.

10. The TGF-β delivery composition according to claim 9 wherein the polymer carrier material is a polymer microsphere comprising poly (lactide-coglycolide) copolymer.

11. The TGF-β delivery composition according to claim 9 or 10 wherein the TGF-β is selected from the group consisting of mature, precursor, or latent forms of TGF-β1, TGF-β2, TGF-β3, TGF-β4, TGF-β5, or bone morphogenic protein.

12. The TGF-β delivery composition according to claim 9 or 10 wherein the TGF-β is mature biologically active TGF-β1.

13. The TGF-β delivery composition according to claim 9 or 10 wherein the TGF-β is mature biologically active TGF-5β.

14. An improved metallic surgical implant for orthopedic application comprising a porous titanium fiber surface which has been modified to incorporate a TGF-β1 delivery composition according to claim 1 or 9.

15. The improved metallic surgical implant according to claim 14 prepared by a method comprising:
(a) coating the porous titanium fiber surface of the implant with a hydroxyapatite/tricalcium phosphate preparation by plasma spraying onto the titanium fiber surface;
(b) immersing and soaking the coated implant in a TGF-β1 solution such that the TGF-β is adsorbed onto the hydroxyapatite/tricalcium phosphate.

**16.** The improved metallic surgical implant according to claim 14 prepared by a method comprising:
a) coating the porous titanium fiber surface of the implant with a hydroxyapatite/tricalcium phosphate preparation by plasma spraying onto the titanium fiber surface;
b) applying a TGF-β1 delivery composition according to claim 1 or 9 directly onto the HA/TCP coated metallic surgical implant.

Fig. 1

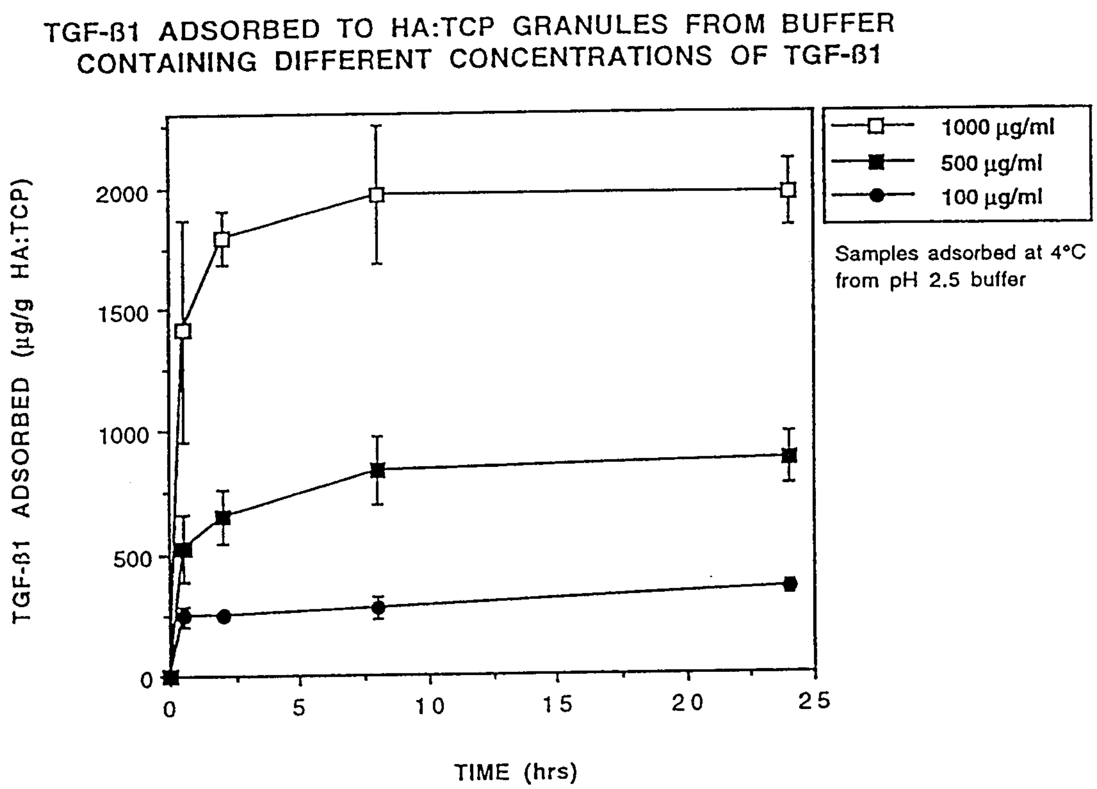
TGF-ß1 ADSORBED TO HA:TCP GRANULES FROM BUFFER CONTAINING DIFFERENT CONCENTRATIONS OF TGF-ß1
TGF-ß1 ADSORBED (µg/g HA:TCP)
2000
1500
1000
500
0
0
5
10
15
20
25
TIME (hrs)
1000 µg/ml
500 µg/ml
100 µg/ml
Samples adsorbed at 4°C from pH 2.5 buffer

Fig. 2

TGF-ß1 ADSORPTION TO HA:TCP AS A FUNCTION OF TGF-ß1 CONCENTRATION

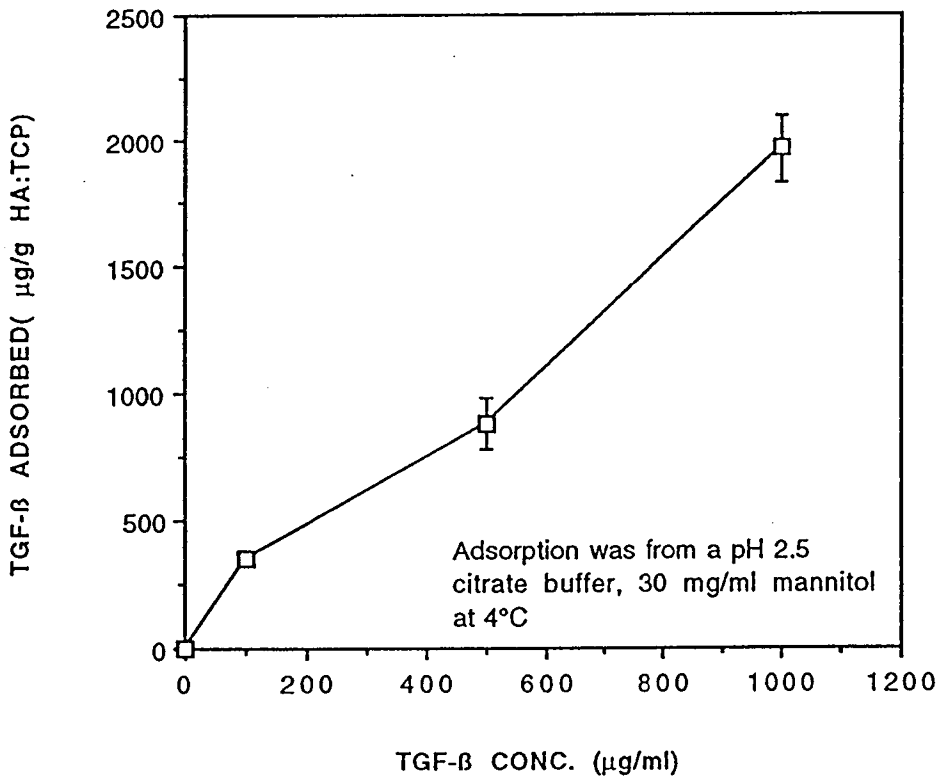

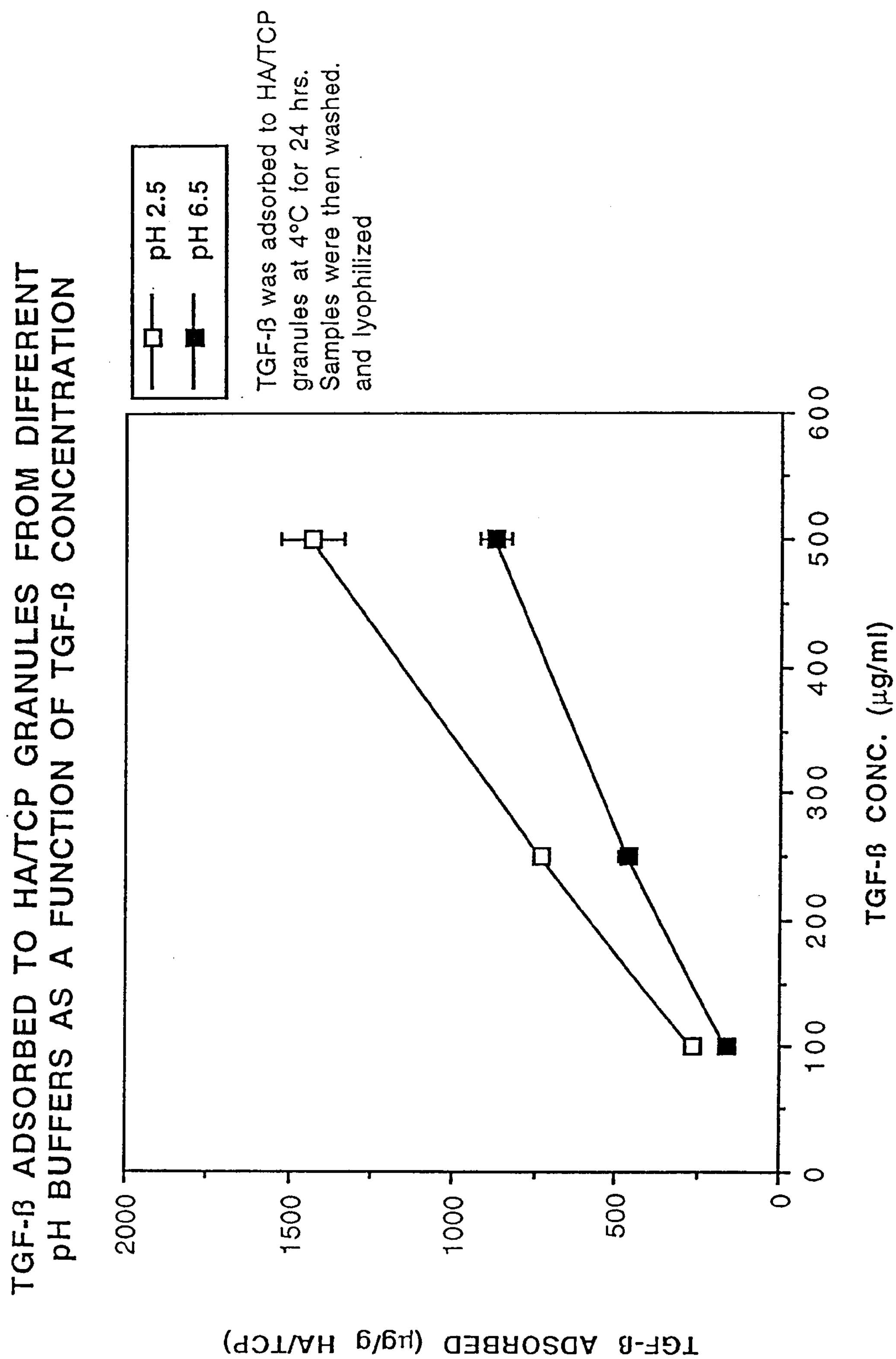
TGF-ß ADSORBED TO HA/TCP GRANULES FROM DIFFERENT pH BUFFERS AS A FUNCTION OF TGF-ß CONCENTRATION
pH 2.5
pH 6.5
TGF-ß was adsorbed to HA/TCP granules at 4°C for 24 hrs. Samples were then washed. and lyophilized
TGF-ß ADSORBED (µg/g HA/TCP)
2000
1500
1000
500
0
0
100
200
300
400
500
600
TGF-ß CONC. (µg/ml)

Fig. 3

Fig. 4

TGF-ß ADSORBED TO HA/TCP GRANULES FROM pH 2.5 BUFFER
AS A FUNCTION OF TGF-ß CONCENTRATION

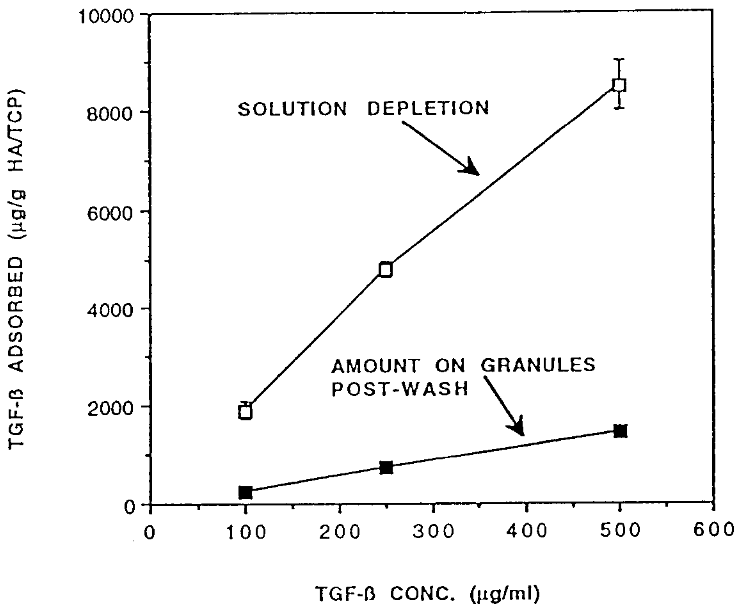


A.

TGF-ß ADSORBED TO HA/TCP GRANULES FROM pH 6.5 BUFFER
AS A FUNCTION OF TGF-ß CONCENTRATION

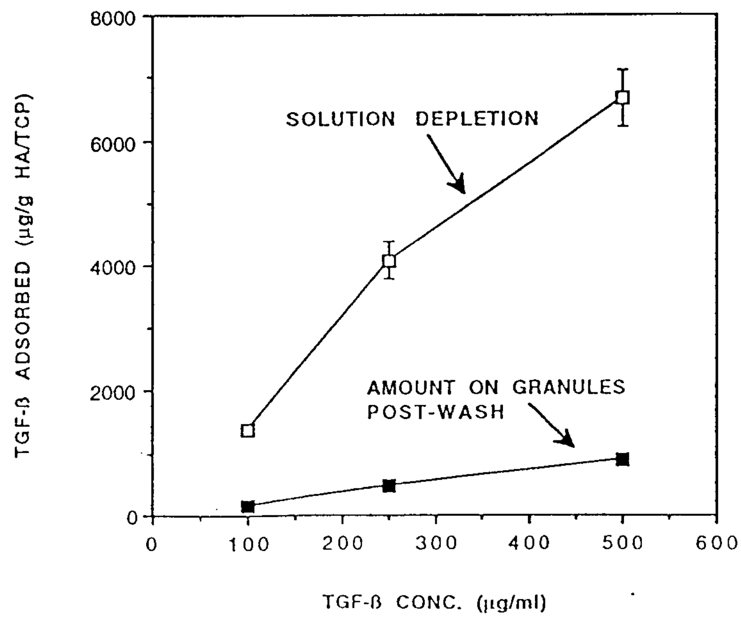


B.

Fig. 5

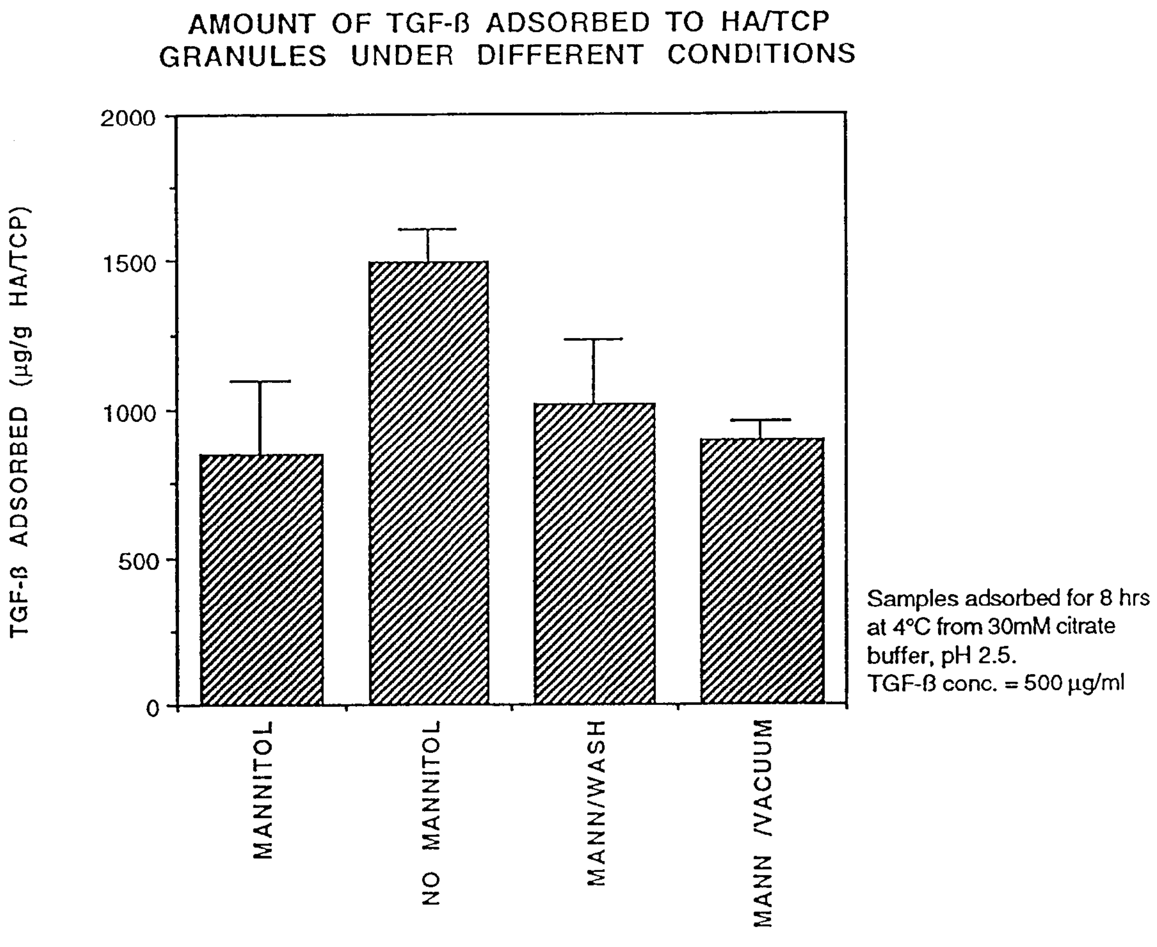
AMOUNT OF TGF-ß ADSORBED TO HA/TCP GRANULES UNDER DIFFERENT CONDITIONS
TGF-ß ADSORBED (µg/g HA/TCP)
2000
1500
1000
500
0
MANNITOL
NO MANNITOL
MANN/WASH
MANN /VACUUM
Samples adsorbed for 8 hrs at 4°C from 30mM citrate buffer, pH 2.5.
TGF-ß conc. = 500 µg/ml

Fig. 6

A.

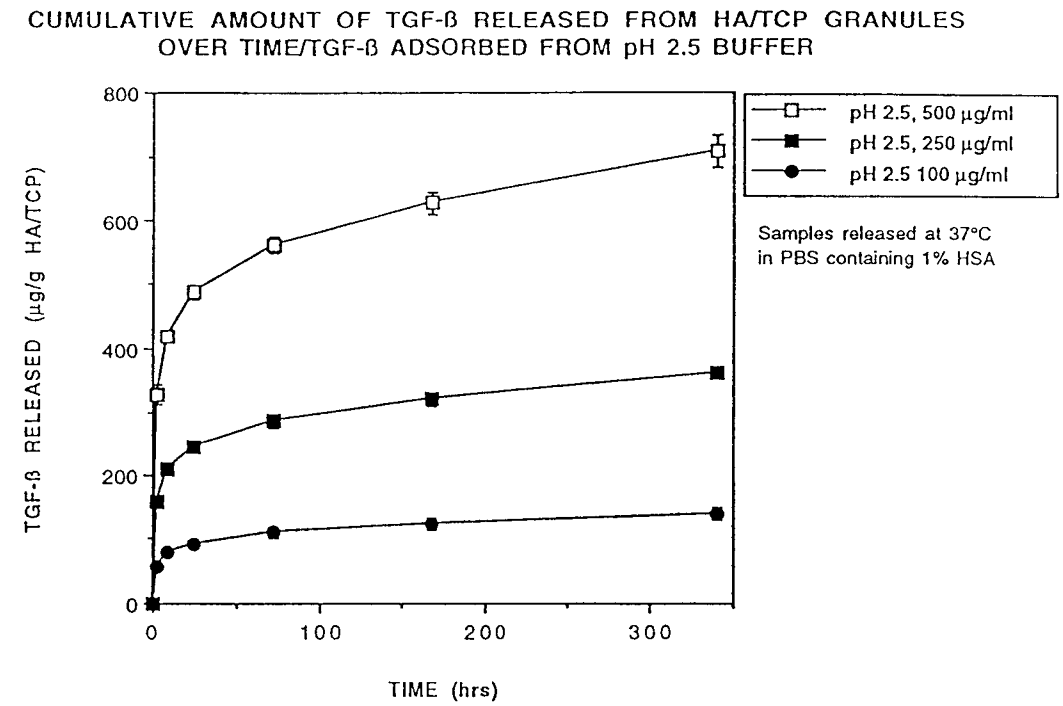
CUMULATIVE AMOUNT OF TGF-ß RELEASED FROM HA/TCP GRANULES
OVER TIME/TGF-ß ADSORBED FROM pH 2.5 BUFFER
TGF-ß RELEASED (µg/g HA/TCP)
800
600
400
200
0
0
100
200
300
TIME (hrs)
pH 2.5, 500 µg/ml
pH 2.5, 250 µg/ml
pH 2.5 100 µg/ml
Samples released at 37°C
in PBS containing 1% HSA

B.

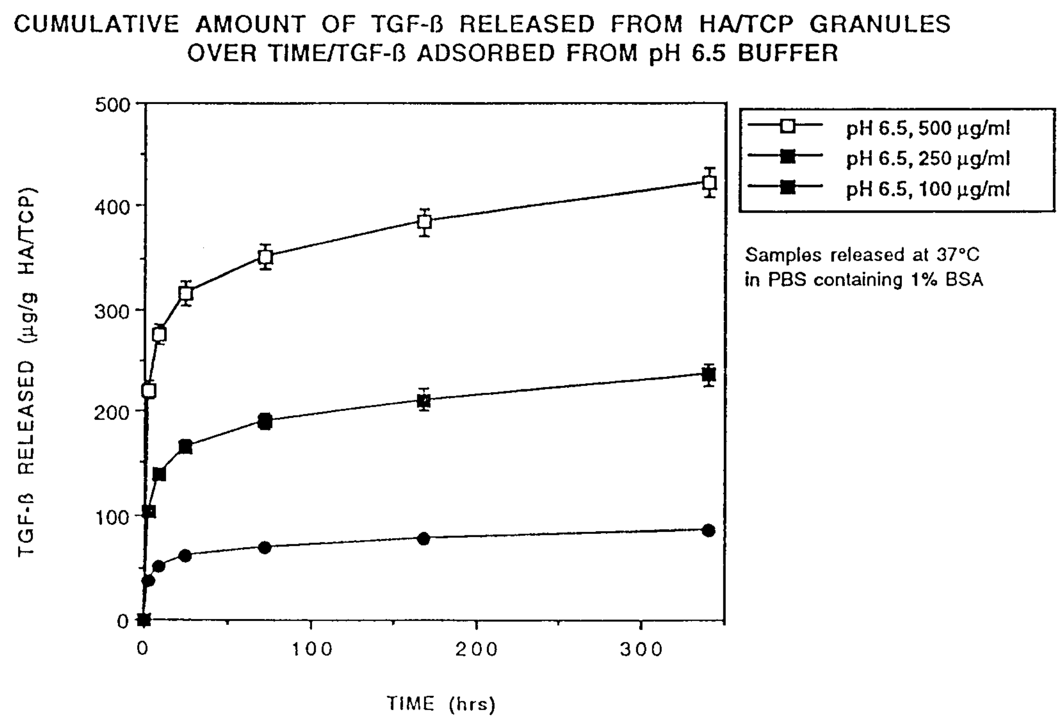
CUMULATIVE AMOUNT OF TGF-ß RELEASED FROM HA/TCP GRANULES
OVER TIME/TGF-ß ADSORBED FROM pH 6.5 BUFFER
TGF-ß RELEASED (µg/g HA/TCP)
500
400
300
200
100
0
0
100
200
300
TIME (hrs)
pH 6.5, 500 µg/ml
pH 6.5, 250 µg/ml
pH 6.5, 100 µg/ml
Samples released at 37°C
in PBS containing 1% BSA

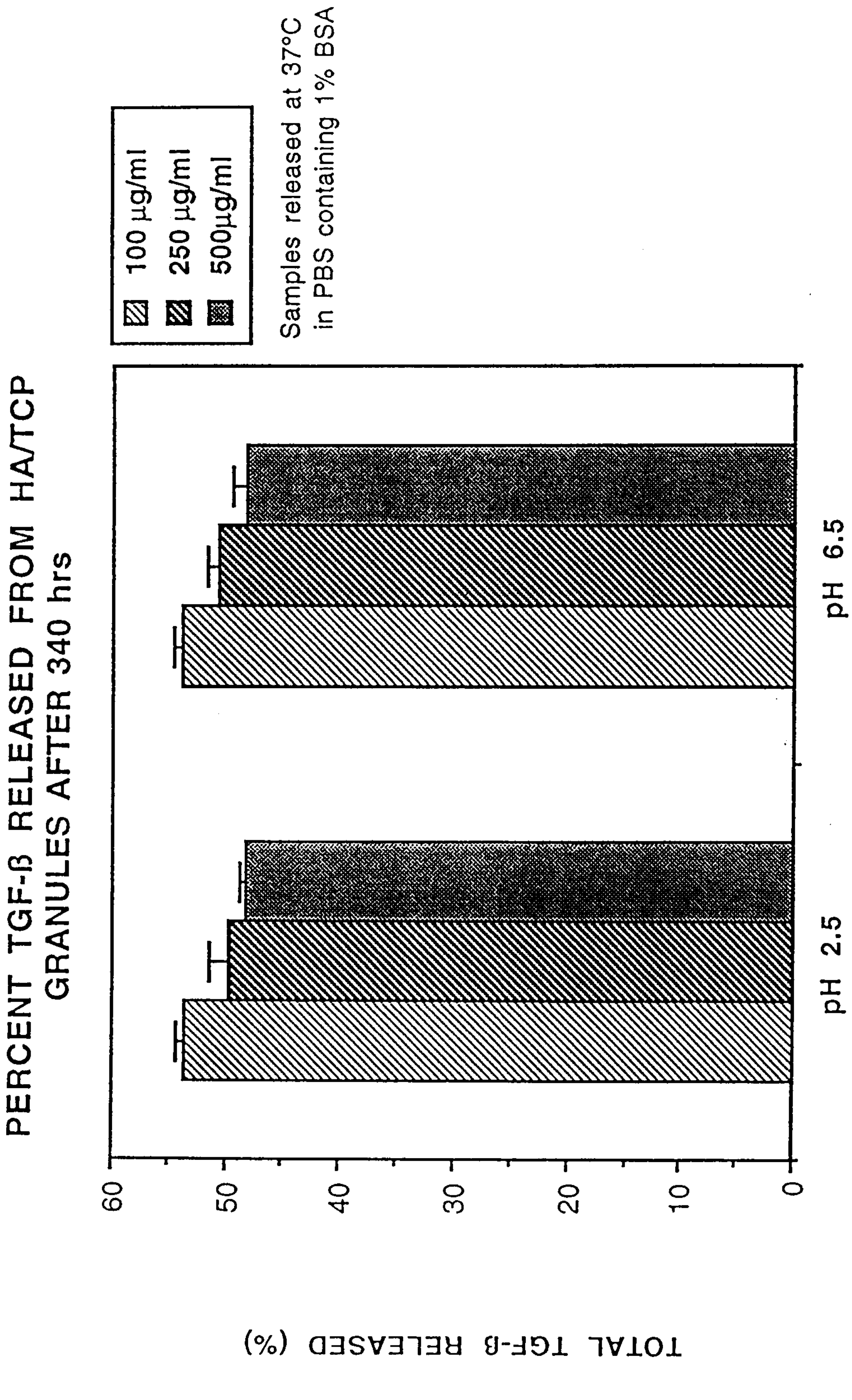
PERCENT TGF-ß RELEASED FROM HA/TCP
GRANULES AFTER 340 hrs
100 µg/ml
250 µg/ml
500µg/ml
Samples released at 37°C
in PBS containing 1% BSA
TOTAL TGF-ß RELEASED (%)
0
10
20
30
40
50
60
pH 2.5
pH 6.5

Fig. 7

Fig. 8

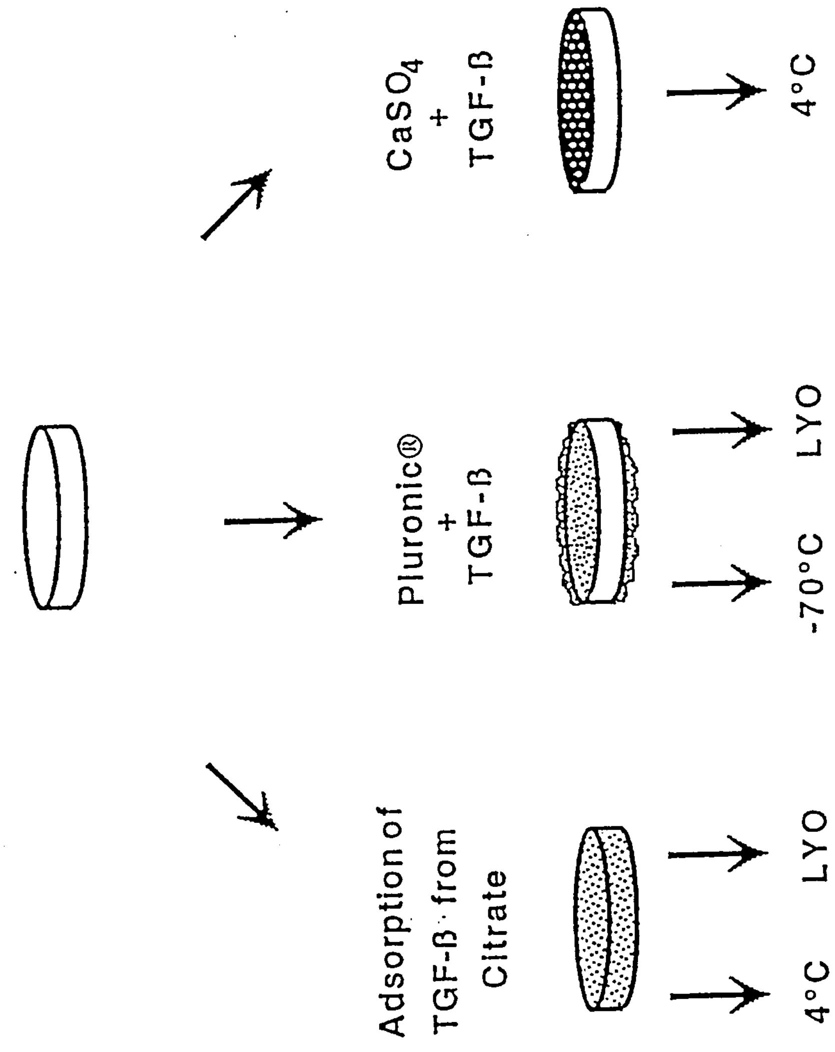
$CaSO_4$ + TGF-ß
4°C
Pluronic® + TGF-ß
-70°C
LYO
Adsorption of TGF-ß from Citrate
4°C
LYO

Fig. 9

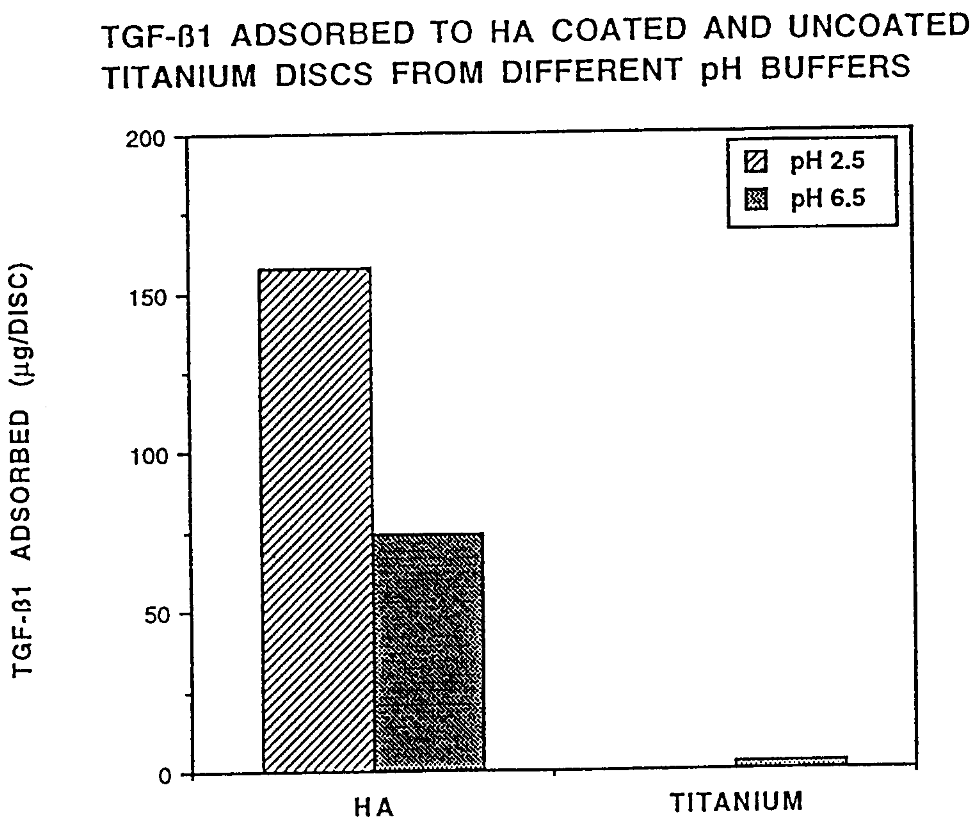
TGF-ß1 ADSORBED TO HA COATED AND UNCOATED TITANIUM DISCS FROM DIFFERENT pH BUFFERS
TGF-ß1 ADSORBED (µg/DISC)
200
150
100
50
0
pH 2.5
pH 6.5
HA
TITANIUM

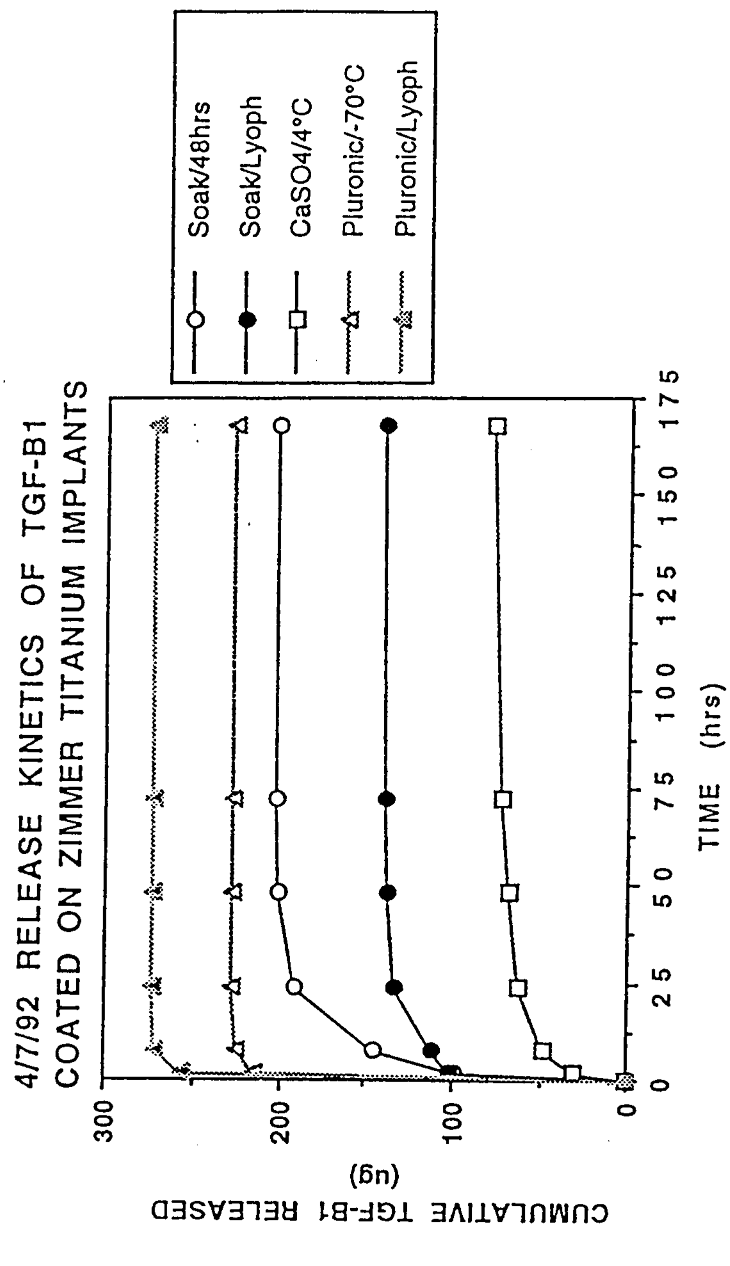
4/7/92 RELEASE KINETICS OF TGF-B1
COATED ON ZIMMER TITANIUM IMPLANTS
Soak/48hrs
Soak/Lyoph
CaSO4/4°C
Pluronic/-70°C
Pluronic/Lyoph
CUMULATIVE TGF-B1 RELEASED
(ug)
300
200
100
0
0
25
50
75
100
125
150
175
TIME (hrs)

Fig. 10

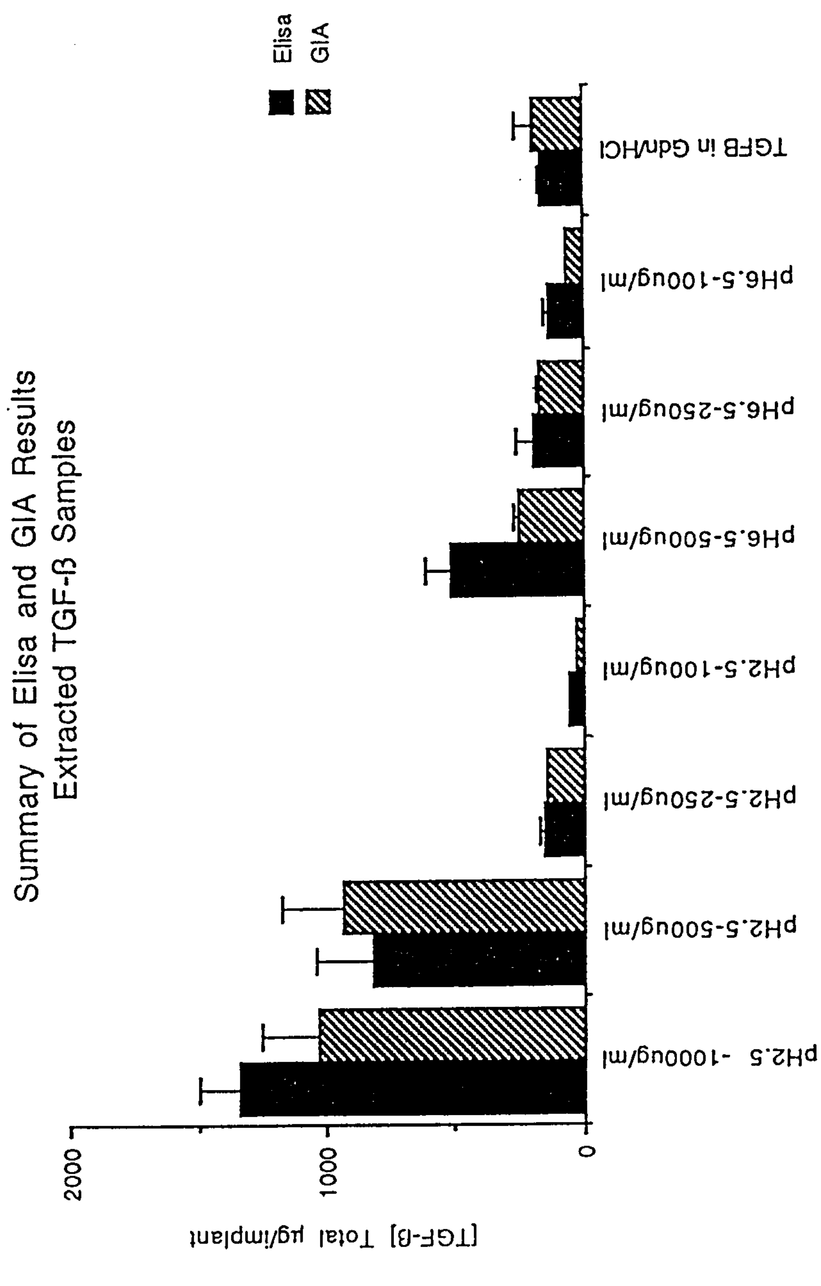
Summary of Elisa and GIA Results
Extracted TGF-β Samples
Elisa
GIA
[TGF-β] Total pg/implant
2000
1000
0
pH2.5 -1000ug/ml
pH2.5-500ug/ml
pH2.5-250ug/ml
pH2.5-100ug/ml
pH6.5-500ug/ml
pH6.5-250ug/ml
pH6.5-100ug/ml
TGFB in Gdn/HCl

Fig. 11

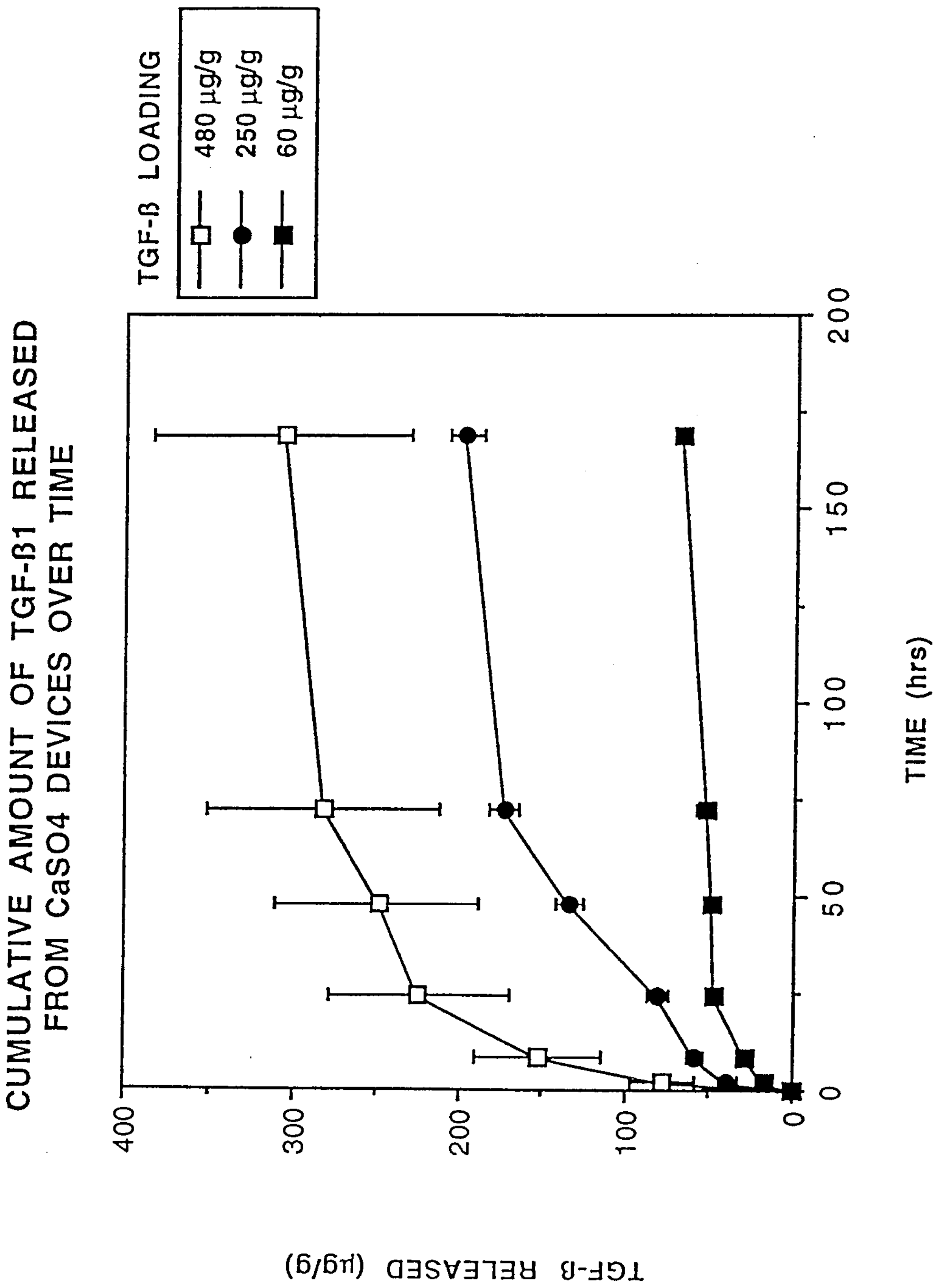
CUMULATIVE AMOUNT OF TGF-ß1 RELEASED FROM CaSO4 DEVICES OVER TIME
TGF-ß LOADING
480 µg/g
250 µg/g
60 µg/g
TGF-ß RELEASED (µg/g)
0
100
200
300
400
0
50
100
150
200
TIME (hrs)

Fig. 12

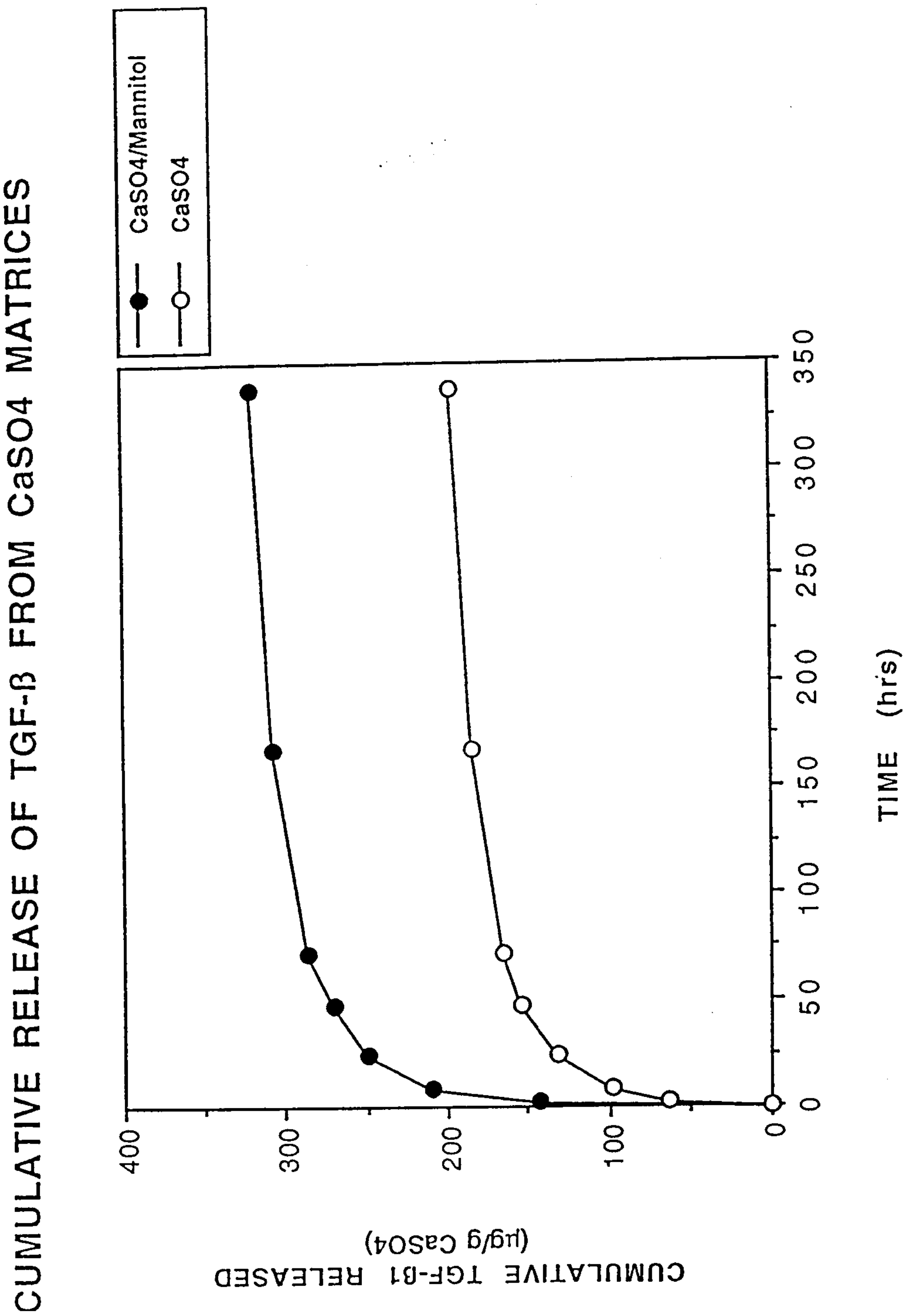
CUMULATIVE RELEASE OF TGF-β FROM CaSO4 MATRICES
CaSO4/Mannitol
CaSO4
CUMULATIVE TGF-β1 RELEASED
(µg/g CaSO4)
0
100
200
300
400
TIME (hrs)
0
50
100
150
200
250
300
350

Fig. 13

# TGF-β1 RELEASE KINETICS FROM DIFFERENT MW PLPG MICROSPHERES

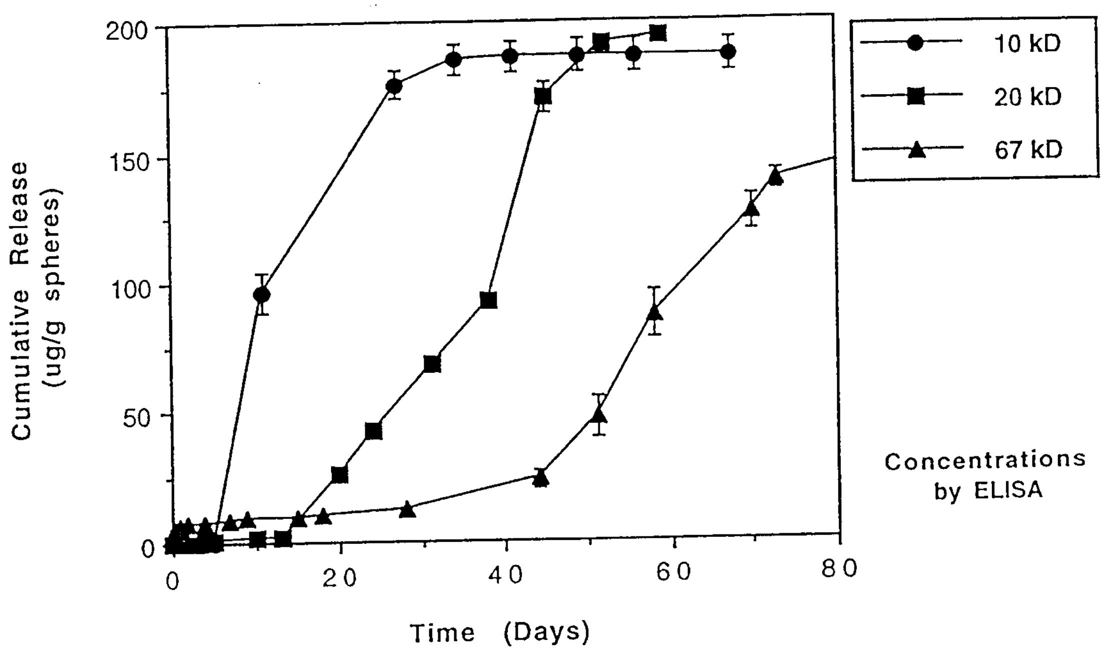


Fig. 14

Fig. 15

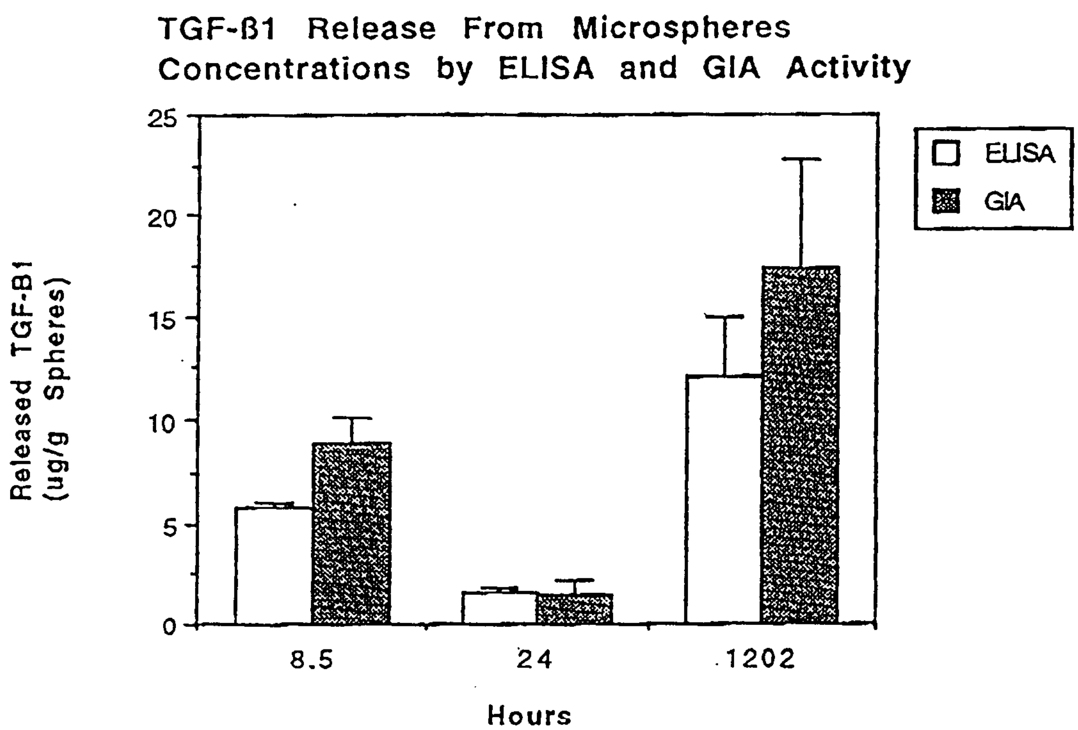
TGF-ß1 Release From Microspheres
Concentrations by ELISA and GIA Activity
Released TGF-B1
(ug/g Spheres)
25
20
15
10
5
0
ELISA
GIA
8.5
24
1202
Hours

European Patent Office

# EUROPEAN SEARCH REPORT

Application Number
EP 94 40 0608

## DOCUMENTS CONSIDERED TO BE RELEVANT

| Category | Citation of document with indication, where appropriate, of relevant passages | Relevant to claim | CLASSIFICATION OF THE APPLICATION (Int.Cl.5) |
|---|---|---|---|
| X | EP-A-0 361 896 (COLLAGEN CORP.)<br>* page 7, line 22 - line 25; claims * | 1-16 | A61L27/00<br>A61L25/00<br>A61K37/02 |
| X | FR-A-2 675 694 (SOUTH AFRICAN MEDICAL RESEARCH COUNCIL.) | 1-3 | |
| X | EP-A-0 395 187 (INTERPORE INT.)<br>* page 8, line 39 - line 44; claims * | 1,2 | |
| X | US-A-4 563 489 (MARSHALL R. URIST.)<br>* claims * | 9,11 | |
| X | EP-A-0 375 127 (GENENTECH, INC.)<br>* examples 1-3 * | 9 | |
| Y | WO-A-90 15586 (MASSACHUSETTS INSTITUTE OF TECHNOLOGY)<br>* claims * | 1-16 | |
| Y | EP-A-0 159 089 (STICHTING BIOMATERIALS SCIENCE CENTER,VU, "BSC-VU")<br>* claims * | 1-16 | TECHNICAL FIELDS SEARCHED (Int.Cl.5) |
| Y | EP-A-0 530 804 (SHAW, ROBERT FRANCIS.)<br>* column 9, line 10 - line 23 * | 1-16 | A61L<br>A61K |

The present search report has been drawn up for all claims

| Place of search | Date of completion of the search | Examiner |
|---|---|---|
| THE HAGUE | 11 July 1994 | ESPINOSA, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)